# EUROPEAN PATENT APPLICATION

(11) **EP 3 932 425 A1**
(43) Date of publication of application: **05.01.2022**
(21) Application number: 20763207.6
(22) Date of filing: 28.02.2020
(51) Int. Cl.: A61K 39/395, A61K 45/00, A61P 35/00, A61P 43/00, A61K 31/519

(54) **CANCER THERAPY USING 3,5-DISUBSTITUTED BENZENE ALKYNYL COMPOUND AND IMMUNE CHECKPOINT INHIBITOR**

(30) Priority: 28.02.2019 JP 2019035603; 18.06.2019 JP 2019112619
(71) Applicant: Taiho Pharmaceutical Co., Ltd., Chiyoda-ku Tokyo 101-8444 (JP)
(72) Inventor: YONEKURA, Kazuhiko, Tokyo 101-8444 (JP); HIRAI, Hiroshi, Tokyo 101-8444 (JP); MATSUOKA, Kazuaki, Tokushima-shi, Tokushima 771-0194 (JP)
(74) Representative: Hoffmann Eitle
(86) International application number: PCT/JP2020/008527
(87) International publication number: WO 2020/175704

(57) **Abstract**

The problem to be solved by the present disclosure is to provide a novel combination therapy using (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, the combination therapy exhibiting an excellent antitumor effect on cancer patients with resistance to immune checkpoint inhibitors. The present disclosure provides an antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient, the antitumor agent being administered in combination with an immune checkpoint inhibitor, except for pembrolizumab, to a cancer patient with resistance to immune checkpoint inhibitors.

## Description

### Technical Field

### Cross-Reference to Related Applications

This application claims priority based on Japanese Patent Application No. 2019-035603 filed on February 28, 2019, and Japanese Patent Application No. 2019-112619 filed on June 18, 2019, the entire contents of which are incorporated herein by reference. The present disclosure relates to an antitumor agent, an antitumor effect enhancer, and a kit preparation.

### Background Art

Fibroblast growth factors (FGFs) are expressed in various tissues, and are one of the growth factors that regulate cell proliferation and differentiation. The physiological activity of the FGFs is mediated by fibroblast growth factor receptors (FGFRs), which are specific cell surface receptors. FGFRs belong to a receptor protein tyrosine kinase family, and comprise an extracellular ligand-binding domain, a single transmembrane domain, and an intracellular tyrosine kinase domain. Four types of FGFRs (FGFR1, FGFR2, FGFR3, and FGFR4) have been heretofore identified. FGFRs bind to FGFs to form dimers, and are activated by phosphorylation. Activation of the receptors induces mobilization and activation of specific downstream signal transduction molecules, thereby developing physiological functions. Some reports have been made about the relationship between aberrant FGF/FGFR signaling and various human tumors. Aberrant activation of FGF/FGFR signaling in human tumors is considered to be attributable to overexpression of FGFRs and/or gene amplification, gene mutation, chromosomal translocation, insertion, and inversion, gene fusion, or an autocrine or paracrine mechanism by overproduction of FGFs (ligands) (NPL 1, 2, 3, and 4).

On the other hand, the development of cancer immunotherapy has progressed as a new cancer treatment method.

Activation of the adaptive immune response is initiated by the binding of antigenic peptide-MHC complexes to T-cell receptors (TCRs). This binding is further defined by costimulation or coinhibition by binding between the costimulatory molecule B7 family and their receptor CD28 family. That is, in order for T cells to activate an antigen specifically, two characteristic signal transduction events are required, and T cells that have received only antigen stimulation without costimulation from the B7 family enter a state of anergy, and immune tolerance is induced.

Taking advantage of this mechanism to suppress the activation of antigen-specific T cells, cancer cells escape from the immune surveillance system and continue to grow. Therefore, it is considered to be effective for cancer treatment to induce antitumor immune responses in the bodies of cancer patients by strengthening costimulation and blocking coinhibition, and to prevent tumor immune escape. Various proposals have been made for cancer immunotherapy targeting costimulatory molecules (stimulatory costimulatory molecules) or coinhibitory molecules (inhibitory costimulatory molecules) (NPL 5). For example, nivolumab (human IgG4 monoclonal antibody against human PD-1) is used for the treatment of malignant melanoma etc. as an immune checkpoint inhibitor that activates T cells by inhibiting the binding of PD-1 with its ligands (PD-L1 and PD-L2) (PTL 1 and NPL 6). Further, as an immune checkpoint inhibitor that is different from nivolumab in terms of antibody type, pembrolizumab is used for the treatment of malignant melanoma, non-small cell lung cancer, etc. (NPL 6).

(S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one, which is a 3,5-disubstituted benzene alkynyl compound, or a salt thereof is known as an FGFR inhibitor. Combinations of FGFR inhibitors and other antitumor agents have been reported so far (PTL 2 and PTL 3). With regard to combined administration of an FGFR inhibitor and an immune checkpoint inhibitor, for example, PTL 4 discloses a method using an FGFR inhibitor and an anti-PD-1 antibody or an anti-PD-Ll antibody in combination.

Many cases have been reported regarding immune checkpoint inhibitors, it has been clarified that continued administration results in resistance to drugs, and the mechanism thereof has been studied (NPL 9, NPL 10, and NPL 11). As an example of the use of an immune checkpoint inhibitor and an FGFR inhibitor, for example, a phase II trial (NCT02365597) of erdafitinib (JNJ-42756493) for urothelial cancer has reported a test in which FGFR inhibitors were administered to subjects who had been pretreated with PD-1 or PD-L1. Further, a combination test of vofatamab, which is an anti-FGFR3 antibody, and pembrolizumab is in progress (NCT03123055). As continued administration, reports have been made on the combined use of everolimus and pazopanib, which is a multikinase inhibitor that has an FGFR inhibitory effect, the combined use of vofatamab and docetaxel, and continued administration of atezolizumab (NPL 12).

### Citation List

### Patent Literature

PTL 1: WO2004/004771
PTL 2: WO2013/108809
PTL 3: WO2017/150725
PTL 4: WO2016/161239

### Non-patent Literature

NPL 1: Nat. Rev. Cancer, 10, 116-129 (2010)
NPL 2: J. Clin. Oncol., 24, 3664-3671 (2006)
NPL 3: Mol. Cancer Res., 3, 655-667 (2005)
NPL 4: Cancer Res., 70, 2085-2094 (2010)
NPL 5: Nat. Rev. Cancer, 12, 252-264 (2012)
NPL 6: N. Engl. J. Med., 366, 2443-2454 (2012)
NPL 7: The Breast, 37, 126-133 (2018)
NPL 8: Oncotarget, 8, 16052-16074 (2017)
NPL 9: Cell, 168, 707-723 (2017)
NPL 10: N. Engl. J. Med., 375, 819-829 (2016)
NPL 11: Sci. Transl. Med. 9, eaal 3604 (2017)
NPL 12: JCO Precision Oncology (DOI: 10.1200/P0.18.00117), July 24, 2018
NPL 13: Cellular Physiology and Biochemistry, 33, pp. 633-645 (2014)
NPL 14: Frontiers in Immunology, 9, 2018, Article 1310.doi:10.3389/fimmu.2018.01310

### Summary of Invention

### Technical Problem

An object of the present disclosure is to provide a novel combination therapy that has excellent antitumor effects for cancer patients who are resistant to immune checkpoint inhibitors by using (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof.

### Solution to Problem

In view of such circumstances, the present inventors found that the above problems can be solved by the combined use of (S)-1-(3-(4-amino-3-((3, 5-dimethoxyphenyl)ethynyl)-1H-pyrazolo[3,4-d]pyrimidin-1-yl)pyrrolidin-1-yl)prop-2-en-1-one (hereinafter also referred to as "compound 1"), which is a compound having FGFR inhibitory activity, or a salt thereof with an immune checkpoint inhibitor.

Therefore, the present disclosure provides the following Items 1 to 77.

Item 1. An antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient, the antitumor agent being administered in combination with an immune checkpoint inhibitor, except for pembrolizumab, to a cancer patient with resistance to immune checkpoint inhibitors.

Item 2. The antitumor agent according to Item 1, wherein the immune checkpoint inhibitor is at least one member selected from the group consisting of a PD-1 pathway antagonist, an ICOS pathway agonist, a CTLA-4 pathway antagonist, and a CD28 pathway agonist.

Item 3. The antitumor agent according to Item 1 or 2, wherein the immune checkpoint inhibitor is a PD-1 pathway antagonist.

Item 4. The antitumor agent according to Item 2 or 3, wherein the PD-1 pathway antagonist is at least one member selected from the group consisting of an anti-PD-1 antibody, an anti-PD-Ll antibody, and an anti-PD-L2 antibody.

Item 5. The antitumor agent according to any one of Items 2 to 4, wherein the PD-1 pathway antagonist is an anti-PD-1 antibody.

Item 6. The antitumor agent according to Item 4 or 5, wherein the anti-PD-1 antibody is nivolumab, cemiplimab, spartalizumab, tislelizumab, BI754091, dostarlimab, sasanlimab, MGA-012, cetrelimab, AGEN-2034, zimberelimab, camrelizumab, budigalimab, or balstilimab; and preferably nivolumab.

Item 7. The antitumor agent according to any one of Items 2 to 4, wherein the PD-1 pathway antagonist is an anti-PD-L1 antibody.

Item 8. The antitumor agent according to Item 7, wherein the anti-PD-Ll antibody is atezolizumab, durvalumab, or avelumab.

Item 9. The antitumor agent according to Item 2, wherein the CTLA-4 pathway antagonist is an anti-CTLA-4 antibody.

Item 10. The antitumor agent according to Item 9, wherein the anti-CTLA-4 antibody is ipilimumab or tremelimumab.

Item 11. The antitumor agent according to any one of Items 1 to 10, wherein a tumor has an aberration in the FGFR pathway.

Item 12. The antitumor agent according to Item 11, wherein the aberration in the FGFR pathway is at least one member selected from the group consisting of FGFR overexpression, FGFR genetic aberration, and aberrant FGFR signaling.

Item 13. The antitumor agent according to any one of Items 1 to 12, wherein treatment with the antitumor agent results in a sustained response in an individual after cessation of the treatment.

Item 14. The antitumor agent according to any one of Items 1 to 13, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is used before, simultaneously with, or after the immune checkpoint inhibitor.

Item 15. The antitumor agent according to any one of Items 1 to 14, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is used continuously or intermittently.

Item 16. The antitumor agent according to any one of Items 1 to 15, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and the immune checkpoint inhibitor are administered in treatment with one therapeutic regimen.

Item 17. The antitumor agent according to any one of Items 1 to 6 and 11 to 16, wherein for a tumor with resistance to immune checkpoint inhibitors, the antitumor agent is used to administer (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof once a day at a dose selected from the group consisting of 4 mg, 8 mg, 12 mg, 16 mg, and 20 mg, and to administer nivolumab at a dose selected from the group consisting of 1 mg/kg in 3-week intervals, 2 mg/kg in 3-week intervals, 3 mg/kg in 3-week intervals, 80 mg in 3-week intervals, 240 mg in 3-week intervals, 1 mg/kg in 2-week intervals, 2 mg/kg in 2-week intervals, 3 mg/kg in 2-week intervals, 80 mg in 2-week intervals, and 240 mg in 2-week intervals.

Item 18. The antitumor agent according to Item 17, wherein for a tumor with resistance to immune checkpoint inhibitors, the antitumor agent is used to administer (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof once a day at a dose selected from the group consisting of 8 mg, 12 mg, 16 mg, and 20 mg, and to administer nivolumab at a dose selected from the group consisting of 240 mg in 2-week intervals and 240 mg in 3-week intervals.

Item 19. The antitumor agent according to Item 17, wherein for a tumor with resistance to immune checkpoint inhibitors, the antitumor agent is used to administer (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof once a day at a dose selected from the group consisting of 8 mg, 12 mg, 16 mg, and 20 mg, and to administer nivolumab at a dose of 240 mg in 2-week intervals.

Item 20. The antitumor agent according to Item 17, wherein for a tumor with resistance to immune checkpoint inhibitors, the antitumor agent is used to administer (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof once a day at a dose selected from the group consisting of 4 mg, 8 mg, 12 mg, 16 mg, and 20 mg, and to administer nivolumab at a dose of 80 mg four times in 3-week intervals and then from the fifth time 240 mg in 2-week intervals.

Item 21. A pharmaceutical composition comprising an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as active ingredients.

Item 22. The pharmaceutical composition according to Item 21, for the treatment of cancer with resistance to immune checkpoint inhibitors.

Item 23. Use of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the manufacture of an antitumor agent for treating cancer with resistance to immune checkpoint inhibitors.

Item 24. Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the manufacture of an antitumor agent to be administered in combination with an immune checkpoint inhibitor, except for pembrolizumab, to a patient with resistance to immune checkpoint inhibitors.

Item 25. Use of an immune checkpoint inhibitor, except for pembrolizumab, for the manufacture of an antitumor agent to be administered in combination with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof to a patient with resistance to immune checkpoint inhibitors.

Item 26. A combination of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for use in treatment of cancer with resistance to immune checkpoint inhibitors.

Item 27. (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for use in combination therapy with an immune checkpoint inhibitor, except for pembrolizumab, in treatment of cancer with resistance to immune checkpoint inhibitors.

Item 28. An immune checkpoint inhibitor, except for pembrolizumab, for use in combination therapy with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in treatment of cancer with resistance to immune checkpoint inhibitors.

Item 29. Use of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the treatment of cancer with resistance to immune checkpoint inhibitors.

Item 30. Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the treatment of cancer with resistance to immune checkpoint inhibitors by combination therapy with an immune checkpoint inhibitor, except for pembrolizumab.

Item 31. Use of an immune checkpoint inhibitor, except for pembrolizumab, for the treatment of cancer with resistance to immune checkpoint inhibitors by combination therapy with (S)-1-(3-(4-amino-3-((3,5-dimethoxyphenyl)ethynyl)-IH-pyrazolo[3,4-d]pyrimidin-1-yl)pyrrolidin-1-yl)prop-2-en-1-one or a salt thereof.

Item 32. A method for treating cancer with resistance to immune checkpoint inhibitors, the method comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, and a therapeutically effective amount of an immune checkpoint inhibitor, except for pembrolizumab, to a human in need thereof.

Item 33. An antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient, the antitumor agent being administered in combination with an immune checkpoint inhibitor, except for pembrolizumab, to a cancer patient who has not been administered immune checkpoint inhibitors.

Item 34. A pharmaceutical composition comprising an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as active ingredients, for the treatment of cancer in a cancer patient who has not been administered immune checkpoint inhibitors.

Item 35. The pharmaceutical composition according to Item 34, for the treatment of cancer with resistance to immune checkpoint inhibitors.

Item 36. Use of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the manufacture of an antitumor agent for treating cancer in a cancer patient who has not been administered immune checkpoint inhibitors.

Item 37. Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the manufacture of an antitumor agent to be administered in combination with an immune checkpoint inhibitor, except for pembrolizumab, to a cancer patient who has not been administered immune checkpoint inhibitors.

Item 38. Use of an immune checkpoint inhibitor, except for pembrolizumab, for the manufacture of an antitumor agent to be administered in combination with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof to a cancer patient who has not been administered immune checkpoint inhibitors.

Item 39. A combination of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for use in treatment of cancer in a cancer patient who has not been administered immune checkpoint inhibitors.

Item 40. (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for use in combination therapy with an immune checkpoint inhibitor, except for pembrolizumab, in treatment of cancer in a cancer patient who has not been administered immune checkpoint inhibitors.

Item 41. An immune checkpoint inhibitor, except for pembrolizumab, for use in combination therapy with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in treatment of cancer in a cancer patient who has not been administered immune checkpoint inhibitors.

Item 42. Use of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the treatment of cancer in a cancer patient who has not been administered immune checkpoint inhibitors.

Item 43. Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the treatment of cancer in a cancer patient who has not been administered immune checkpoint inhibitors by combination therapy with an immune checkpoint inhibitor, except for pembrolizumab.

Item 44. Use of an immune checkpoint inhibitor, except for pembrolizumab, for the treatment of cancer in a cancer patient who has not been administered immune checkpoint inhibitors by combination therapy with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof.

Item 45. A method for treating cancer in a cancer patient who has not been administered immune checkpoint inhibitors, the method comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, and a therapeutically effective amount of an immune checkpoint inhibitor, except for pembrolizumab, to a human in need thereof.

Item 46. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 45, wherein a tumor has an aberration in the FGFR pathway.

Item 47. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to Item 46, wherein the aberration in the FGFR pathway is at least one member selected from the group consisting of FGFR overexpression, FGFR genetic aberration, and aberrant FGFR signaling.

Item 48. An antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient, the antitumor agent being administered in combination with an immune checkpoint inhibitor, except for pembrolizumab, to a cancer patient with resistance to immune checkpoint inhibitors and without aberrations in the FGFR pathway.

Item 49. A pharmaceutical composition comprising an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as active ingredients, for the treatment of cancer without aberrations in the FGFR pathway.

Item 50. The pharmaceutical composition according to Item 49, for the treatment of cancer with resistance to immune checkpoint inhibitors.

Item 51. Use of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the manufacture of an antitumor agent for treating cancer without aberrations in the FGFR pathway.

Item 52. Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the manufacture of an antitumor agent to be administered in combination with an immune checkpoint inhibitor, except for pembrolizumab, to a cancer patient without aberrations in the FGFR pathway.

Item 53. Use of an immune checkpoint inhibitor, except for pembrolizumab, for the manufacture of an antitumor agent to be administered in combination with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof to a cancer patient without aberrations in the FGFR pathway.

Item 54. A combination of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for use in treatment of cancer without aberrations in the FGFR pathway.

Item 55. (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for use in combination therapy with an immune checkpoint inhibitor, except for pembrolizumab, in treatment of cancer without aberrations in the FGFR pathway.

Item 56. An immune checkpoint inhibitor, except for pembrolizumab, for use in combination therapy with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in treatment of cancer without aberrations in the FGFR pathway.

Item 57. Use of an immune checkpoint inhibitor, except for pembrolizumab, and (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the treatment of cancer without aberrations in the FGFR pathway.

Item 58. Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof for the treatment of cancer without aberrations in the FGFR pathway by combination therapy with an immune checkpoint inhibitor, except for pembrolizumab.

Item 59. Use of an immune checkpoint inhibitor, except for pembrolizumab, for the treatment of cancer without aberrations in the FGFR pathway by combination therapy with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof.

Item 60. A method for treating cancer without aberrations in the FGFR pathway, the method comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, and a therapeutically effective amount of an immune checkpoint inhibitor, except for pembrolizumab, to a human in need thereof.

Item 61. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 18 to 29, wherein the immune checkpoint inhibitor is at least one member selected from the group consisting of a PD-1 pathway antagonist, an ICOS pathway agonist, a CTLA-4 pathway antagonist, and a CD28 pathway agonist.

Item 62. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 61, wherein the immune checkpoint inhibitor is a PD-1 pathway antagonist.

Item 63. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to Item 61 or 62, wherein the PD-1 pathway antagonist is at least one member selected from the group consisting of an anti-PD-1 antibody, an anti-PD-Ll antibody, and an anti-PD-L2 antibody.

Item 64. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 61 to 63, wherein the PD-1 pathway antagonist is an anti-PD-1 antibody.

Item 65. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to Item 63 or 64, wherein the anti-PD-1 antibody is nivolumab, cemiplimab, spartalizumab, tislelizumab, BI754091, dostarlimab, sasanlimab, MGA-012, cetrelimab, AGEN-2034, zimberelimab, camrelizumab, budigalimab, or balstilimab; and preferably nivolumab.

Item 66. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 61 to 63, wherein the PD-1 pathway antagonist is an anti-PD-Ll antibody.

Item 67. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to Item 64, wherein the anti-PD-Ll antibody is atezolizumab, durvalumab, or avelumab.

Item 68. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to Item 61, wherein the CTLA-4 pathway antagonist is an anti-CTLA-4 antibody.

Item 69. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to Item 68, wherein the anti-CTLA-4 antibody is ipilimumab or tremelimumab.

Item 70. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 69, wherein treatment with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof and the immune checkpoint inhibitor results in a sustained response in an individual after cessation of the treatment.

Item 71. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 70, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof is used before, simultaneously with, or after the immune checkpoint inhibitor.

Item 72. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 71, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof is used continuously or intermittently.

Item 73. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 71, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof and the immune checkpoint inhibitor are administered in treatment with one therapeutic regimen.

Item 74. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 65 and 70 to 73, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof and nivolumab are used in the following manner: to a cancer patient with resistance to immune checkpoint inhibitors, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof is administered once a day at a dose selected from the group consisting of 4 mg, 8 mg, 12 mg, 16 mg, and 20 mg; and nivolumab is administered at a dose selected from the group consisting of 1 mg/kg in 3-week intervals, 2 mg/kg in 3-week intervals, 3 mg/kg in 3-week intervals, 80 mg in 3-week intervals, 240 mg in 3-week intervals, 1 mg/kg in 2-week intervals, 2 mg/kg in 2-week intervals, 3 mg/kg in 2-week intervals, 80 mg in 2-week intervals, and 240 mg in 2-week intervals.

Item 75. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 65 and 70 to 73, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof and nivolumab are used in the following manner: to a cancer patient with resistance to immune checkpoint inhibitors, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof is administered once a day at a dose selected from the group consisting of 8 mg, 12 mg, 16 mg, and 20 mg; and nivolumab is administered at a dose selected from the group consisting of 240 mg in 2-week intervals and 240 mg in 3-week intervals.

Item 76. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 65 and 70 to 73, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof and nivolumab are used in the following manner: to a cancer patient with resistance to immune checkpoint inhibitors, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof is administered once a day at a dose selected from the group consisting of 8 mg, 12 mg, 16 mg, and 20 mg; and nivolumab is administered at a dose of 240 mg in 2-week intervals.

Item 77. The composition, use, combination, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, immune checkpoint inhibitor, or method according to any one of Items 21 to 65 and 70 to 73, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof and nivolumab are used in the following manner: to a cancer patient with resistance to immune checkpoint inhibitors, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or the salt thereof is administered once a day at a dose selected from the group consisting of 4 mg, 8 mg, 12 mg, 16 mg, and 20 mg; and nivolumab is administered at a dose of 80 mg four times in 3-week intervals and then from the fifth time 240 mg in 2-week intervals.

### Advantageous Effects of Invention

The present disclosure can provide a novel combination therapy that has excellent antitumor effects for cancer patients who are resistant to immune checkpoint inhibitors by using (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof.

### Brief Description of Drawings

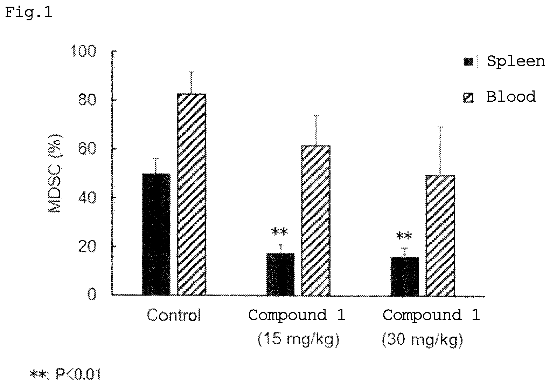

Fig. 1 shows the results of Example 1 (influence of compound 1 on myeloid-derived suppressor cells (MDSCs)).

### Description of Embodiments

The present disclosure relates to the provision of, for example, combined use of a compound having FGFR inhibitory activity and an immune checkpoint inhibitor, except for pembrolizumab, that is, an antitumor agent comprising a compound having FGFR inhibitory activity and being administered in combination with an immune checkpoint inhibitor, except for pembrolizumab, an antitumor effect enhancer comprising a compound having FGFR inhibitory activity, a combination of a compound having FGFR inhibitory activity and an immune checkpoint inhibitor, except for pembrolizumab, and use of a compound having FGFR inhibitory activity in the production of a medicament for the treatment of tumors performed in combination with an immune checkpoint inhibitor, except for pembrolizumab.

In the present disclosure, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one, which exhibits an excellent antitumor effect when used in combination with an immune checkpoint inhibitor, is a disubstituted benzene alkynyl compound having the following structure. In the present specification, this compound is simply referred to as "compound 1." Compound 1 is described as Example Compound 2 in PTL 2 mentioned above. It has been reported that compound 1 or a salt thereof has an excellent FGFR inhibitory effect and inhibits the ability of receptor protein tyrosine kinases (FGFR1, FGFR3, and FGFR4) to phosphorylate tyrosine in the substrate peptide sequence (PTL 2).

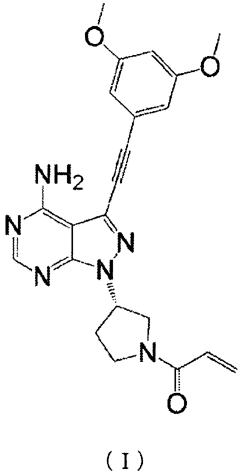

Compound 1 or a pharmaceutically acceptable salt thereof in the present disclosure can be synthesized by any method; for example, it can be synthesized according to the production method described in PTL 2.

In the present disclosure, compound 1 can be used as is or in the form of a pharmaceutically acceptable salt. The pharmaceutically acceptable salt of compound 1 is not particularly limited, and examples include addition salts with inorganic acids such as hydrochloric acid and sulfuric acid, or with organic acids such as acetic acid, citric acid, tartaric acid, and maleic acid; salts with alkali metals such as potassium and sodium; salts with alkaline earth metals such as calcium and magnesium; salts with organic bases such as ammonium salts, ethylamine salts, and arginine salts; and the like.

In the present disclosure, immune checkpoint inhibitors act on immune checkpoint molecules, and have the effects of inducing antitumor immune responses in the bodies of subjects and preventing tumor immune escape.

Examples of such immune checkpoint inhibitors include substances that promote the function of costimulatory molecules (stimulatory costimulatory molecules), and substances that inhibit the function of coinhibitory molecules (inhibitory costimulatory molecules). Examples of immune checkpoint molecules include the B7 family (B7-1, B7-2, PD-L1, PD-L2, etc.), CD28 family (CTLA-4, PD-1, etc.), TNF superfamily (4-1BBL, OX40L), and TNF receptor superfamily (4-1BB, OX40) molecules. Substances that target immune checkpoint molecules can be used as immune checkpoint inhibitors. Examples include PD-1 pathway antagonists, ICOS pathway agonists, CTLA-4 pathway antagonists, CD28 pathway agonists, BTLA pathway antagonists, 4-1BB pathway agonists, and the like.

In the present disclosure, preferred is at least one or more members selected from the group consisting of PD-1 pathway antagonists, ICOS pathway agonists, CTLA-4 pathway antagonists, and CD28 pathway agonists; more preferred is at least one or more members selected from the group consisting of PD-1 pathway antagonists and CTLA-4 pathway antagonists; and even more preferred are PD-1 pathway antagonists.

PD-1 pathway antagonists inhibit immunosuppressive signals of PD-1 expressed on T cells and its ligand PD-L1 or PD-L2. Examples include, but are not limited to, anti-PD-1 antibodies, anti-PD-Ll antibodies, anti-PD-L2 antibodies, PD-1 extracellular domains, PD-L1 extracellular domains, PD-L2 extracellular domains, PD-1-Ig (fusion protein of a PD-1 extracellular domain and the FC region of Ig), PD-L1-Ig, PD-L2-Ig, PD-1 siRNA, PD-L1 siRNA, PD-L2 siRNA, and the like. Preferred are anti-PD-1 antibodies, anti-PD-Ll antibodies, or anti-PD-L2 antibodies; and more preferred are anti-PD-1 antibodies or anti-PD-L1 antibodies. Particularly preferred among these are anti-PD-1 antibodies.

Further, CTLA-4 pathway antagonists inhibit immunosuppressive signals of CTLA-4 expressed on T cells and its ligands B7-1 (CD80) and B7-2 (CD86). Preferable examples include anti-CTLA-4 antibodies, CTLA-4 extracellular domains, CTLA-4-Ig (fusion protein of a CTLA-4 extracellular domain and the FC region of Ig), anti-B7-1 (CD80) antibodies, or andi-B7-2 (CD86) antibodies; and more preferred are anti-CTLA-4 antibodies or CTLA-4-Ig. Particularly preferred among these are anti-CTLA-4 antibodies.

Examples of these antibodies include immunoglobulins (IgA, IgD, IgE, IgG, IgM, IgY, etc.), Fab fragments, F(ab')₂ fragments, single-chain antibody fragments (scFv), single-domain antibodies, and Diabody (Nat. Rev. Immunol., 6:343-357, 2006). These include monoclonal antibodies and polyclonal antibodies, such as human antibodies, humanized antibodies, chimeric antibodies, mouse antibodies, llama antibodies, and chicken antibodies. In the present disclosure, immunoglobulins (preferably IgG etc.) and the like are preferable. Further, in the present disclosure, human antibodies or humanized antibodies are preferable. In the present disclosure, monoclonal antibodies are preferable.

Preferred is a humanized IgG monoclonal antibody or a human IgG monoclonal antibody.

In the present disclosure, preferable anti-PD-1 antibodies include nivolumab, cemiplimab, spartalizumab, tislelizumab, BI754091, dostarlimab, sasanlimab, MGA-012, cetrelimab, AGEN-2034, zimberelimab, camrelizumab, budigalimab, balstilimab, and the like

In the present disclosure, preferable anti-PD-Ll antibodies include atezolizumab, durvalumab, avelumab, lodapolimab, BGB-A333, and the like.

In the present disclosure, preferable anti-CTLA-4 antibodies include ipilimumab, tremelimumab, and the like.

In the present disclosure, preferable CTLA-4-Ig includes abatacept and the like.

These agonists or antagonists can be produced by a generally known production method.

Further, anti-PD-1 antibodies have already been sold or will be sold as nivolumab; anti-PD-Ll antibodies as atezolizumab, durvalumab, or avelumab; anti-CTLA-4 antibodies as ipilimumab or tremelimumab; and CTLA-4-Ig as abatacept. These can also be used.

In the present disclosure, the immune checkpoint inhibitors can be used singly or in combination of two or more.

In the present disclosure, when two or more immune checkpoint inhibitors are used, for example, an anti-PD-1 antibody, an anti-CTLA-4 antibody, and like immune checkpoint inhibitors can be used in combination, or bispecific antibodies that can bind to different immune checkpoint molecules can also be used. Examples of bispecific antibodies include XmAb20717 (PD-1 × CTLA-4), which can bind to both PD-1 and CTLA-4.

The treatment in the present disclosure includes procedures performed for the purpose of curing or ameliorating diseases, or for the purpose of suppressing disease progression or relapse or alleviating the symptoms. The treatment includes administration of drugs before or after surgical procedure, or administration of drugs during, before, or after radiation therapy.

The therapeutically effective amounts of antibodies and compound 1 or a salt thereof that block the interaction with immune checkpoint molecules are not limited, and depend on several factors, including the stage and severity of cancer, as well as other factors related to the patient's health. Persons skilled in the art would know how to determine the therapeutically effective amounts.

An embodiment of the present disclosure is used for tumors having aberrations in the FGFR pathway. In an embodiment, the FGFR is selected from the group consisting of FGFR1, FGFR2, FGFR3, and FGFR4.

In an embodiment, the tumors to which the present disclosure is to be applied are preferably tumors having aberrations in the FGFR pathway. The aberrations in the FGFR pathway include FGFR overexpression, FGFR genetic aberration, and aberrant FGFR signaling.

In an embodiment of the present disclosure, the presence of FGF/FGFR aberrations does not matter; however, the administration subject is preferably one having aberrations in the FGFR pathway. Compound 1 can promote the antitumor effect of immune checkpoint inhibitors even in cancer types without FGFR genetic aberrations. The susceptibility of cancer to immune checkpoint inhibitors is known to be also affected by the tumor microenvironment. The microenvironment includes, but is not limited to, cell groups controlled by FGF/FGFR pathway activation, such as cancer-associated fibroblasts (CAFs). These cell groups are known to diminish the susceptibility of cancer to immune checkpoint inhibitors. The administration of compound 1 is considered to cause changes in the microenvironment, including inactivation of CAFs, thereby promoting the effects of immune checkpoint inhibitors on cancer.

Examples of FGFR overexpression include gene expression and high expression of gene product proteins. The expression of FGFR can be detected by methods known to persons skilled in the art. Gene expression and high expression of gene product proteins can be detected by known methods, such as methods using antibodies (immunohistochemistry, enzyme immunoassay (ELISA), flow cytometry, immunoblotting, etc.), methods using nucleic acids (*in situ* hybridization, PCR, Northern blotting, etc.), and methods based on a common principle of these methods. The detection device can be any known device (GeneChip, microarray, etc.).

An embodiment of the present disclosure is used for tumors having FGFR genetic aberrations. Genetic aberrations in the FGFR pathway include gene amplification, gene mutation, chromosomal translocation, insertion, and inversion, gene fusion, genetic rearrangement, etc. Some of the FGFR genetic aberrations in tumors have already been reported in documents available to persons skilled in the art (NPL 7 and NPL 8). FGFR genetic aberrations can be detected by methods known to persons skilled in the art, for example, pyrosequence, DNA sequencing including next-generation sequencing (NGS), PCR-based methods including allele-specific PCR chain reactions, microarray-based comparative genomic hybridization (aCGH), fluorescence *in situ* hybridization (FISH), and chromogenic *in situ* hybridization (CISH).

Further, an embodiment of the present disclosure is used for tumors with activated FGFR signaling. FGFRs bind to FGFs to form dimers and are activated by phosphorylation, which induces mobilization and activation of specific downstream signal transduction molecules, thereby developing physiological functions.

Activation of FGFR signaling can be detected by methods known to persons skilled in the art. Signaling activation can be detected, for example, by detecting FGFs, which are substrates for FGFRs; by detecting direct phosphorylation state of FGFRs, which are phosphorylated enzymes, or biological activity including enzymatic activity; by detecting phosphorylation state of intracellular substrates and intracellular proteins existing in the FGFR signaling cascade downstream, or biological activity including enzymatic activity; or by detecting gene products or gene transcripts.

In another embodiment, the tumors to which the present disclosure is to be applied also include tumors having no aberrations in the FGFR pathway.

In the present disclosure, preferred examples of the dose of compound 1 or a salt thereof per day on the day of administration include 4 mg to 160 mg, 4 mg to 24 mg, 12 to 24 mg, 16 to 24 mg, 20 mg, and the like. More specifically, preferred examples of the number of doses and the dose per day on the day of administration include once a day, 4 mg, 8 mg, 12 mg, 16 mg, 20 mg, and the like. In another embodiment, preferred examples of the number of doses and the dose per day on the day of administration include 8 mg, 12 mg, 16 mg, 20 mg, and the like. In another embodiment, preferred examples of the number of doses and the dose per day on the day of administration include 12 mg, 16 mg, 20 mg, and the like. In another embodiment, preferred examples of the number of doses and the dose per day on the day of administration include 20 mg and the like.

In an embodiment of the present disclosure, in the case of a cancer type for which a stronger effect is desired (e.g., brain tumor), the dose of compound 1 or a salt thereof includes a dose larger than 20 mg once a day.

When the dose of compound 1 or a salt thereof per day is administered intermittently, the dose of compound 1 or a pharmaceutically acceptable salt thereof per day is, for example, about 2 to 1000 mg, preferably 10 to 500 mg, more preferably 20 to 200 mg, and even more preferably 50 to 160 mg.

Regarding the administration schedule, compound 1 or a salt thereof can be administered every day or intermittently.

In the present disclosure, "administered every day" may be an administration schedule based on a cycle in which dosing is performed for 21 days every day (one cycle), and a drug holiday may be provided as each cycle ends.

In the present disclosure, "administered intermittently" is not particularly limited as long as the conditions of at least twice a week and an administration interval of at least one day between dosing (the number of days between a certain day of administration and the next day of administration) are satisfied.

Examples include an administration schedule based on a 1-week cycle, in which compound 1 or a pharmaceutically acceptable salt thereof is administered at least twice every one to three days per cycle (with an interval between a certain day of administration and the next day of administration of 1 to 3 days), and this cycle is performed once or repeated twice or more;
an administration schedule based on a 14-day cycle, in which compound 1 or a salt thereof is administered 4 to 7 times every one to three days per cycle (with an interval between a certain day of administration and the next day of administration of 1 to 3 days), and this cycle is performed once or repeated twice or more;
an administration schedule based on a 14-day cycle, in which, among 14 days contained in one cycle, compound 1 or a salt thereof is administered on days 1, 4, 8, and 11;
an administration schedule based on a 14-day cycle, in which, among 14 days contained in one cycle, compound 1 or a salt thereof is administered on days 1, 3, 5, 7, 9, 11, and 13; and
an administration schedule based on a 14-day cycle, in which, among 14 days contained in one cycle, compound 1 or a salt thereof is administered on days 1, 3, 5, 8, 10, and 12.

In an embodiment of the present disclosure, an example of the administration schedule is such that 160 mg of compound 1 or a pharmaceutically acceptable salt thereof is administered once a day on days 1, 3, 5, 8, 10, and 12. The dose of compound 1 or a salt thereof can be reduced to 120 mg, 80 mg, 56 mg, 36 mg, 24 mg, 16 mg, or 8 mg.

In the present disclosure, in terms of enhancing the antitumor effect of an immune checkpoint inhibitor by compound 1 or a salt thereof, the daily dose of the immune checkpoint inhibitor on the day of administration is preferably 30 to 100%, more preferably 50 to 100%, even more preferably 70 to 100%, still more preferably 80 to 100%, further still more preferably 90 to 100%, and further still more preferably 100%, of the recommended dose in the case of administering the immune checkpoint inhibitor alone.

The preferred dose of the immune checkpoint inhibitor when administered alone varies depending on the type of drug etc. Examples include 1 to 4 mg/kg (body weight) per dose, 2 to 3 mg/kg (body weight) per dose, 2 mg/kg (body weight) per dose, 3 mg/kg (body weight) per dose, and the like.

Specifically, the preferred dose of nivolumab when administered alone is, for example, 1 to 3 mg/kg (body weight) per dose or 80 to 240 mg per dose, 2 to 3 mg/kg (body weight) per dose or 160 to 240 mg per dose, 2 mg/kg (body weight) per dose, 3 mg/kg (body weight) per dose or 240 mg per dose.

In another embodiment, the preferred dose of nivolumab when administered alone is, for example, 80 to 480 mg per dose, 80 mg per dose, 240 mg per dose, or 480 mg per dose.

The preferred dose of cemiplimab when administered alone is, for example, 350 mg per dose.

The preferred dose of atezolizumab when administered alone is, for example, 840 to 1680 mg per dose, or 840, 1200, or 1680 mg per dose.

The preferred dose of ipilimumab when administered alone is, for example, 1.0 to 10 mg/kg (body weight) per dose, or 1.0, 3.0, or 10 mg/kg (body weight) per dose.

The preferred dose of durvalumab when administered alone is, for example, 5.0 to 10 mg/kg (body weight) per dose or 10 mg/kg (body weight) per dose.

The preferred dose of avelumab when administered alone is, for example, 10 mg/kg (body weight) per dose or 800 mg per dose.

In the present disclosure, the preferred dose of nivolumab per day on the day of administration is, for example, 40 to 480 mg per dose, or 80, 240, or 480 mg per dose. Further, the administration interval of nivolumab is preferably 2 to 4 weeks, more preferably 2 to 3 weeks, and even more preferably 2 weeks.

Further, in a preferred embodiment, the dose and administration interval of nivolumab are such that 80 mg or 240 mg of nivolumab is administered four times in 3-week intervals, and then from the fifth time, 240 mg of nivolumab is administered in two-week intervals.

In the present disclosure, the preferred dose of cemiplimab per day on the day of administration is, for example, 175 to 350 mg per dose or 350 mg per dose. The administration interval of cemiplimab is preferably 2 to 4 weeks, more preferably 2 to 3 weeks, and even more preferably 2 weeks.

In the present disclosure, the preferred dose of atezolizumab per day on the day of administration is, for example, 420 to 1680 mg per dose, or 840, 1200, or 1680 mg. The administration interval of atezolizumab is preferably 2 to 4 weeks, more preferably 2 to 3 weeks, and even more preferably 3 weeks.

In the present disclosure, the preferred dose of ipilimumab per day on the day of administration is, for example, 0.5 to 10 mg/kg (body weight) per dose, or 1.0, 3.0, or 10 mg/kg (body weight) per dose. The administration interval of ipilimumab is preferably 2 to 4 weeks, more preferably 2 to 3 weeks, and even more preferably 3 weeks.

In the present disclosure, the preferred dose of durvalumab per day on the day of administration is, for example, 5.0 to 10 mg/kg (body weight) per dose or 10 mg/kg (body weight) per dose. The administration interval of durvalumab is preferably 2 to 4 weeks, more preferably 2 to 3 weeks, and even more preferably 2 weeks.

In the present disclosure, the preferred dose of avelumab per day on the day of administration is, for example, 5.0 to 10 g/kg (body weight) per dose or 10 mg/kg (body weight) per dose. Further, in another embodiment, the preferred dose of avelumab per day on the day of administration is, for example, 400 to 800 mg per dose or 800 mg per dose. The administration interval of avelumab is preferably 2 to 4 weeks, more preferably 2 to 3 weeks, and even more preferably 2 weeks.

Examples of the dose of compound 1 or a salt thereof of the present disclosure per day on the day of administration, and the dose of nivolumab per day, or a preferred combination of usage and dosage include the following:
4 mg to 160 mg of compound 1 or a salt thereof of the present disclosure and 1 to 4 mg/kg (body weight) per dose of nivolumab.
4 mg to 160 mg of compound 1 or a salt thereof of the present disclosure and 1 to 3 mg/kg (body weight) per dose of nivolumab.
4 mg to 160 mg of compound 1 or a salt thereof of the present disclosure and 2 to 3 mg/kg (body weight) per dose of nivolumab.
4 mg to 160 mg of compound 1 or a salt thereof of the present disclosure and 1 mg/kg (body weight) per dose of nivolumab.
4 mg to 160 mg of compound 1 or a salt thereof of the present disclosure and 2 mg/kg (body weight) per dose of nivolumab.
4 mg to 160 mg of compound 1 or a salt thereof of the present disclosure and 3 mg/kg (body weight) per dose of nivolumab.
4 mg to 160 mg of compound 1 or a salt thereof of the present disclosure and 80 to 240 mg of nivolumab per dose.
4 mg to 160 mg of compound 1 or a salt thereof of the present disclosure and 80 to 480 mg of nivolumab per dose.
4 mg to 160 mg of compound 1 or a salt thereof of the present disclosure and 160 to 480 mg of nivolumab per dose.
4 mg to 160 mg of compound 1 or a salt thereof of the present disclosure and 80 mg of nivolumab per dose.
4 mg to 160 mg of compound 1 or a salt thereof of the present disclosure and 240 mg of nivolumab per dose.
4 mg to 160 mg of compound 1 or a salt thereof of the present disclosure and 480 mg of nivolumab per dose.
4 mg to 160 mg of compound 1 or a salt thereof of the present disclosure and 1 mg/kg of nivolumab in 2-week intervals.
4 mg to 160 mg of compound 1 or a salt thereof of the present disclosure and 2 mg/kg of nivolumab in 2-week intervals.
4 mg to 160 mg of compound 1 or a salt thereof of the present disclosure and 3 mg/kg of nivolumab in 2-week intervals.
4 mg to 160 mg of compound 1 or a salt thereof of the present disclosure and 1 mg/kg of nivolumab in 3-week intervals.
4 mg to 160 mg of compound 1 or a salt thereof of the present disclosure and 2 mg/kg of nivolumab in 3-week intervals.
4 mg to 160 mg of compound 1 or a salt thereof of the present disclosure and 3 mg/kg of nivolumab in 3-week intervals.
4 mg to 160 mg of compound 1 or a salt thereof of the present disclosure and 1 mg/kg of nivolumab in 4-week intervals.
4 mg to 160 mg of compound 1 or a salt thereof of the present disclosure and 2 mg/kg of nivolumab in 4-week intervals.
4 mg to 160 mg of compound 1 or a salt thereof of the present disclosure and 3 mg/kg of nivolumab in 4-week intervals.
4 mg to 160 mg of compound 1 or a salt thereof of the present disclosure and 80 mg of nivolumab in 2-week intervals.
4 mg to 160 mg of compound 1 or a salt thereof of the present disclosure and 240 mg of nivolumab in 2-week intervals.
4 mg to 160 mg of compound 1 or a salt thereof of the present disclosure and 480 mg of nivolumab in 2-week intervals.
4 mg to 160 mg of compound 1 or a salt thereof of the present disclosure and 80 mg of nivolumab in 3-week intervals.
4 mg to 160 mg of compound 1 or a salt thereof of the present disclosure and 240 mg of nivolumab in 3-week intervals.
4 mg to 160 mg of compound 1 or a salt thereof of the present disclosure and 480 mg of nivolumab in 3-week intervals.
4 mg to 160 mg of compound 1 or a salt thereof of the present disclosure and 80 mg of nivolumab in 4-week intervals.
4 mg to 160 mg of compound 1 or a salt thereof of the present disclosure and 240 mg of nivolumab in 4-week intervals.
4 mg to 160 mg of compound 1 or a salt thereof of the present disclosure and 480 mg of nivolumab in 4-week intervals.
4 mg to 160 mg of compound 1 or a salt thereof of the present disclosure and 80 mg of nivolumab four times in 3-week intervals, and then from the fifth time, 240 mg of nivolumab in 2-week intervals.
4 mg to 160 mg of compound 1 or a salt thereof of the present disclosure and 240 mg of nivolumab four times in 3-week intervals, and then from the fifth time, 240 mg of nivolumab in 2-week intervals.
4 mg to 160 mg of compound 1 or a salt thereof of the present disclosure and 80 mg of nivolumab four times in 3-week intervals, and then from the fifth time, 480 mg of nivolumab in 4-week intervals.
4 mg to 160 mg of compound 1 or a salt thereof of the present disclosure and 240 mg of nivolumab four times in 3-week intervals, and then from the fifth time, 480 mg of nivolumab in 4-week intervals.
4 mg to 24 mg of compound 1 or a salt thereof of the present disclosure and 1 to 4 mg/kg (body weight) per dose of nivolumab.
4 mg to 24 mg of compound 1 or a salt thereof of the present disclosure and 1 to 3 mg/kg (body weight) per dose of nivolumab.
4 mg to 24 mg of compound 1 or a salt thereof of the present disclosure and 2 to 3 mg/kg (body weight) per dose of nivolumab.
4 mg to 24 mg of compound 1 or a salt thereof of the present disclosure and 1 mg/kg (body weight) per dose of nivolumab.
4 mg to 24 mg of compound 1 or a salt thereof of the present disclosure and 2 mg/kg (body weight) per dose of nivolumab.
4 mg to 24 mg of compound 1 or a salt thereof of the present disclosure and 3 mg/kg (body weight) per dose of nivolumab.
4 mg to 24 mg of compound 1 or a salt thereof of the present disclosure and 80 to 240 mg of nivolumab per dose.
4 mg to 24 mg of compound 1 or a salt thereof of the present disclosure and 80 to 480 mg of nivolumab per dose.
4 mg to 24 mg of compound 1 or a salt thereof of the present disclosure and 160 to 480 mg of nivolumab per dose.
4 mg to 24 mg of compound 1 or a salt thereof of the present disclosure and 80 mg of nivolumab per dose.
4 mg to 24 mg of compound 1 or a salt thereof of the present disclosure and 240 mg of nivolumab per dose.
4 mg to 24 mg of compound 1 or a salt thereof of the present disclosure and 480 mg of nivolumab per dose.
4 mg to 24 mg of compound 1 or a salt thereof of the present disclosure and 1 mg/kg of nivolumab in 2-week intervals.
4 mg to 24 mg of compound 1 or a salt thereof of the present disclosure and 2 mg/kg of nivolumab in 2-week intervals.
4 mg to 24 mg of compound 1 or a salt thereof of the present disclosure and 3 mg/kg of nivolumab in 2-week intervals.
4 mg to 24 mg of compound 1 or a salt thereof of the present disclosure and 1 mg/kg of nivolumab in 3-week intervals.
4 mg to 24 mg of compound 1 or a salt thereof of the present disclosure and 2 mg/kg of nivolumab in 3-week intervals.
4 mg to 24 mg of compound 1 or a salt thereof of the present disclosure and 3 mg/kg of nivolumab in 3-week intervals.
4 mg to 24 mg of compound 1 or a salt thereof of the present disclosure and 1 mg/kg of nivolumab in 4-week intervals.
4 mg to 24 mg of compound 1 or a salt thereof of the present disclosure and 2 mg/kg of nivolumab in 4-week intervals.
4 mg to 24 mg of compound 1 or a salt thereof of the present disclosure and 3 mg/kg of nivolumab in 4-week intervals.
4 mg to 24 mg of compound 1 or a salt thereof of the present disclosure and 80 mg of nivolumab in 2-week intervals.
4 mg to 24 mg of compound 1 or a salt thereof of the present disclosure and 240 mg of nivolumab in 2-week intervals.
4 mg to 24 mg of compound 1 or a salt thereof of the present disclosure and 480 mg of nivolumab in 2-week intervals.
4 mg to 24 mg of compound 1 or a salt thereof of the present disclosure and 80 mg of nivolumab in 3-week intervals.
4 mg to 24 mg of compound 1 or a salt thereof of the present disclosure and 240 mg of nivolumab in 3-week intervals.
4 mg to 24 mg of compound 1 or a salt thereof of the present disclosure and 480 mg of nivolumab in 3-week intervals.
4 mg to 24 mg of compound 1 or a salt thereof of the present disclosure and 80 mg of nivolumab in 4-week intervals.
4 mg to 24 mg of compound 1 or a salt thereof of the present disclosure and 240 mg of nivolumab in 4-week intervals.
4 mg to 24 mg of compound 1 or a salt thereof of the present disclosure and 480 mg of nivolumab in 4-week intervals.
4 mg to 24 mg of compound 1 or a salt thereof of the present disclosure and 80 mg of nivolumab four times in 3-week intervals, and then from the fifth time, 240 mg of nivolumab in 2-week intervals.
4 mg to 24 mg of compound 1 or a salt thereof of the present disclosure and 240 mg of nivolumab four times in 3-week intervals, and then from the fifth time, 240 mg of nivolumab in 2-week intervals.
4 mg to 24 mg of compound 1 or a salt thereof of the present disclosure and 80 mg of nivolumab four times in 3-week intervals, and then from the fifth time, 480 mg of nivolumab in 4-week intervals.
4 mg to 24 mg of compound 1 or a salt thereof of the present disclosure and 240 mg of nivolumab four times in 3-week intervals, and then from the fifth time, 480 mg of nivolumab in 4-week intervals.
12 to 24 mg of compound 1 or a salt thereof of the present disclosure and 1 to 4 mg/kg (body weight) per dose of nivolumab.
12 to 24 mg of compound 1 or a salt thereof of the present disclosure and 1 to 3 mg/kg (body weight) per dose of nivolumab.
12 to 24 mg of compound 1 or a salt thereof of the present disclosure and 2 to 3 mg/kg (body weight) per dose of nivolumab.
12 to 24 mg of compound 1 or a salt thereof of the present disclosure and 1 mg/kg (body weight) per dose of nivolumab.
12 to 24 mg of compound 1 or a salt thereof of the present disclosure and 2 mg/kg (body weight) per dose of nivolumab.
12 to 24 mg of compound 1 or a salt thereof of the present disclosure and 3 mg/kg (body weight) per dose of nivolumab.
12 to 24 mg of compound 1 or a salt thereof of the present disclosure and 80 to 240 mg of nivolumab per dose.
12 to 24 mg of compound 1 or a salt thereof of the present disclosure and 80 to 480 mg of nivolumab per dose.
12 to 24 mg of compound 1 or a salt thereof of the present disclosure and 160 to 480 mg of nivolumab per dose.
12 to 24 mg of compound 1 or a salt thereof of the present disclosure and 80 mg of nivolumab per dose.
12 to 24 mg of compound 1 or a salt thereof of the present disclosure and 240 mg of nivolumab per dose.
12 to 24 mg of compound 1 or a salt thereof of the present disclosure and 480 mg of nivolumab per dose.
12 to 24 mg of compound 1 or a salt thereof of the present disclosure and 1 mg/kg of nivolumab in 2-week intervals.
12 to 24 mg of compound 1 or a salt thereof of the present disclosure and 2 mg/kg of nivolumab in 2-week intervals.
12 to 24 mg of compound 1 or a salt thereof of the present disclosure and 3 mg/kg of nivolumab in 2-week intervals.
12 to 24 mg of compound 1 or a salt thereof of the present disclosure and 1 mg/kg of nivolumab in 3-week intervals.
12 to 24 mg of compound 1 or a salt thereof of the present disclosure and 2 mg/kg of nivolumab in 3-week intervals.
12 to 24 mg of compound 1 or a salt thereof of the present disclosure and 3 mg/kg of nivolumab in 3-week intervals.
12 to 24 mg of compound 1 or a salt thereof of the present disclosure and 1 mg/kg of nivolumab in 4-week intervals.
12 to 24 mg of compound 1 or a salt thereof of the present disclosure and 2 mg/kg of nivolumab in 4-week intervals.
12 to 24 mg of compound 1 or a salt thereof of the present disclosure and 3 mg/kg of nivolumab in 4-week intervals.
12 to 24 mg of compound 1 or a salt thereof of the present disclosure and 80 mg of nivolumab in 2-week intervals.
12 to 24 mg of compound 1 or a salt thereof of the present disclosure and 240 mg of nivolumab in 2-week intervals.
12 to 24 mg of compound 1 or a salt thereof of the present disclosure and 480 mg of nivolumab in 2-week intervals.
12 to 24 mg of compound 1 or a salt thereof of the present disclosure and 80 mg of nivolumab in 3-week intervals.
12 to 24 mg of compound 1 or a salt thereof of the present disclosure and 240 mg of nivolumab in 3-week intervals.
12 to 24 mg of compound 1 or a salt thereof of the present disclosure and 480 mg of nivolumab in 3-week intervals.
12 to 24 mg of compound 1 or a salt thereof of the present disclosure and 80 mg of nivolumab in 4-week intervals.
12 to 24 mg of compound 1 or a salt thereof of the present disclosure and 240 mg of nivolumab in 4-week intervals.
12 to 24 mg of compound 1 or a salt thereof of the present disclosure and 480 mg of nivolumab in 4-week intervals.
12 to 24 mg of compound 1 or a salt thereof of the present disclosure and 80 mg of nivolumab four times in 3-week intervals, and then from the fifth time, 240 mg of nivolumab in 2-week intervals.
12 to 24 mg of compound 1 or a salt thereof of the present disclosure and 240 mg of nivolumab four times in 3-week intervals, and then from the fifth time, 240 mg of nivolumab in 2-week intervals.
12 to 24 mg of compound 1 or a salt thereof of the present disclosure and 80 mg of nivolumab four times in 3-week intervals, and then from the fifth time, 480 mg of nivolumab in 4-week intervals.
12 to 24 mg of compound 1 or a salt thereof of the present disclosure and 240 mg of nivolumab four times in 3-week intervals, and then from the fifth time, 480 mg of nivolumab in 4-week intervals.
16 to 24 mg of compound 1 or a salt thereof of the present disclosure and 1 to 4 mg/kg (body weight) per dose of nivolumab.
16 to 24 mg of compound 1 or a salt thereof of the present disclosure and 1 to 3 mg/kg (body weight) per dose of nivolumab.
16 to 24 mg of compound 1 or a salt thereof of the present disclosure and 2 to 3 mg/kg (body weight) per dose of nivolumab.
16 to 24 mg of compound 1 or a salt thereof of the present disclosure and 1 mg/kg (body weight) per dose of nivolumab.
16 to 24 mg of compound 1 or a salt thereof of the present disclosure and 2 mg/kg (body weight) per dose of nivolumab.
16 to 24 mg of compound 1 or a salt thereof of the present disclosure and 3 mg/kg (body weight) per dose of nivolumab.
16 to 24 mg of compound 1 or a salt thereof of the present disclosure and 80 to 240 mg of nivolumab per dose.
16 to 24 mg of compound 1 or a salt thereof of the present disclosure and 80 to 480 mg of nivolumab per dose.
16 to 24 mg of compound 1 or a salt thereof of the present disclosure and 160 to 480 mg of nivolumab per dose.
16 to 24 mg of compound 1 or a salt thereof of the present disclosure and 80 mg of nivolumab per dose.
16 to 24 mg of compound 1 or a salt thereof of the present disclosure and 240 mg of nivolumab per dose.
16 to 24 mg of compound 1 or a salt thereof of the present disclosure and 480 mg of nivolumab per dose.
16 to 24 mg of compound 1 or a salt thereof of the present disclosure and 1 mg/kg of nivolumab in 2-week intervals.
16 to 24 mg of compound 1 or a salt thereof of the present disclosure and 2 mg/kg of nivolumab in 2-week intervals.
16 to 24 mg of compound 1 or a salt thereof of the present disclosure and 3 mg/kg of nivolumab in 2-week intervals.
16 to 24 mg of compound 1 or a salt thereof of the present disclosure and 1 mg/kg of nivolumab in 3-week intervals.
16 to 24 mg of compound 1 or a salt thereof of the present disclosure and 2 mg/kg of nivolumab in 3-week intervals.
16 to 24 mg of compound 1 or a salt thereof of the present disclosure and 3 mg/kg of nivolumab in 3-week intervals.
16 to 24 mg of compound 1 or a salt thereof of the present disclosure and 1 mg/kg of nivolumab in 4-week intervals.
16 to 24 mg of compound 1 or a salt thereof of the present disclosure and 2 mg/kg of nivolumab in 4-week intervals.
16 to 24 mg of compound 1 or a salt thereof of the present disclosure and 3 mg/kg of nivolumab in 4-week intervals.
16 to 24 mg of compound 1 or a salt thereof of the present disclosure and 80 mg of nivolumab in 2-week intervals.
16 to 24 mg of compound 1 or a salt thereof of the present disclosure and 240 mg of nivolumab in 2-week intervals.
16 to 24 mg of compound 1 or a salt thereof of the present disclosure and 480 mg of nivolumab in 2-week intervals.
16 to 24 mg of compound 1 or a salt thereof of the present disclosure and 80 mg of nivolumab in 3-week intervals.
16 to 24 mg of compound 1 or a salt thereof of the present disclosure and 240 mg of nivolumab in 3-week intervals.
16 to 24 mg of compound 1 or a salt thereof of the present disclosure and 480 mg of nivolumab in 3-week intervals.
16 to 24 mg of compound 1 or a salt thereof of the present disclosure and 80 mg of nivolumab in 4-week intervals.
16 to 24 mg of compound 1 or a salt thereof of the present disclosure and 240 mg of nivolumab in 4-week intervals.
16 to 24 mg of compound 1 or a salt thereof of the present disclosure and 480 mg of nivolumab in 4-week intervals.
16 to 24 mg of compound 1 or a salt thereof of the present disclosure and 80 mg of nivolumab four times in 3-week intervals, and then from the fifth time, 240 mg of nivolumab in 2-week intervals.
16 to 24 mg of compound 1 or a salt thereof of the present disclosure and 240 mg of nivolumab four times in 3-week intervals, and then from the fifth time, 240 mg of nivolumab in 2-week intervals.
16 to 24 mg of compound 1 or a salt thereof of the present disclosure and 80 mg of nivolumab four times in 3-week intervals, and then from the fifth time, 480 mg of nivolumab in 4-week intervals.
16 to 24 mg of compound 1 or a salt thereof of the present disclosure and 240 mg of nivolumab four times in 3-week intervals, and then from the fifth time, 480 mg of nivolumab in 4-week intervals.

Examples of the number of doses and the dose of compound 1 or a salt thereof of the present disclosure per day on the day of administration, and the dose of nivolumab per day, or a combination of usage and dosage include the following:
4 mg of compound 1 or a salt thereof of the present disclosure once a day and 1 to 4 mg/kg (body weight) per dose of nivolumab.
4 mg of compound 1 or a salt thereof of the present disclosure once a day and 1 to 3 mg/kg (body weight) per dose of nivolumab.
4 mg of compound 1 or a salt thereof of the present disclosure once a day and 2 to 3 mg/kg (body weight) per dose of nivolumab.
4 mg of compound 1 or a salt thereof of the present disclosure once a day and 1 mg/kg (body weight) per dose of nivolumab.
4 mg of compound 1 or a salt thereof of the present disclosure once a day and 2 mg/kg (body weight) per dose of nivolumab.
4 mg of compound 1 or a salt thereof of the present disclosure once a day and 3 mg/kg (body weight) per dose of nivolumab.
4 mg of compound 1 or a salt thereof of the present disclosure once a day and 80 to 240 mg of nivolumab per dose.
4 mg of compound 1 or a salt thereof of the present disclosure once a day and 80 to 480 mg of nivolumab per dose.
4 mg of compound 1 or a salt thereof of the present disclosure once a day and 160 to 480 mg of nivolumab per dose.
4 mg of compound 1 or a salt thereof of the present disclosure once a day and 80 mg of nivolumab per dose.
4 mg of compound 1 or a salt thereof of the present disclosure once a day and 240 mg of nivolumab per dose.
4 mg of compound 1 or a salt thereof of the present disclosure once a day and 480 mg of nivolumab per dose.
4 mg of compound 1 or a salt thereof of the present disclosure once a day and 1 mg/kg of nivolumab in 2-week intervals.
4 mg of compound 1 or a salt thereof of the present disclosure once a day and 2 mg/kg of nivolumab in 2-week intervals.
4 mg of compound 1 or a salt thereof of the present disclosure once a day and 3 mg/kg of nivolumab in 2-week intervals.
4 mg of compound 1 or a salt thereof of the present disclosure once a day and 1 mg/kg of nivolumab in 3-week intervals.
4 mg of compound 1 or a salt thereof of the present disclosure once a day and 2 mg/kg of nivolumab in 3-week intervals.
4 mg of compound 1 or a salt thereof of the present disclosure once a day and 3 mg/kg of nivolumab in 3-week intervals.
4 mg of compound 1 or a salt thereof of the present disclosure once a day and 1 mg/kg of nivolumab in 4-week intervals.
4 mg of compound 1 or a salt thereof of the present disclosure once a day and 2 mg/kg of nivolumab in 4-week intervals.
4 mg of compound 1 or a salt thereof of the present disclosure once a day and 3 mg/kg of nivolumab in 4-week intervals.
4 mg of compound 1 or a salt thereof of the present disclosure once a day and 80 mg of nivolumab in 2-week intervals.
4 mg of compound 1 or a salt thereof of the present disclosure once a day and 240 mg of nivolumab in 2-week intervals.
4 mg of compound 1 or a salt thereof of the present disclosure once a day and 480 mg of nivolumab in 2-week intervals.
4 mg of compound 1 or a salt thereof of the present disclosure once a day and 80 mg of nivolumab in 3-week intervals.
4 mg of compound 1 or a salt thereof of the present disclosure once a day and 240 mg of nivolumab in 3-week intervals.
4 mg of compound 1 or a salt thereof of the present disclosure once a day and 480 mg of nivolumab in 3-week intervals.
4 mg of compound 1 or a salt thereof of the present disclosure once a day and 80 mg of nivolumab in 4-week intervals.
4 mg of compound 1 or a salt thereof of the present disclosure once a day and 240 mg of nivolumab in 4-week intervals.
4 mg of compound 1 or a salt thereof of the present disclosure once a day and 480 mg of nivolumab in 4-week intervals.
4 mg of compound 1 or a salt thereof of the present disclosure once a day and 80 mg of nivolumab four times in 3-week intervals, and then from the fifth time, 240 mg of nivolumab in 2-week intervals.
4 mg of compound 1 or a salt thereof of the present disclosure once a day and 240 mg of nivolumab four times in 3-week intervals, and then from the fifth time, 240 mg of nivolumab in 2-week intervals.
4 mg of compound 1 or a salt thereof of the present disclosure once a day and 80 mg of nivolumab four times in 3-week intervals, and then from the fifth time, 480 mg of nivolumab in 4-week intervals.
4 mg of compound 1 or a salt thereof of the present disclosure once a day and 240 mg of nivolumab four times in 3-week intervals, and then from the fifth time, 480 mg of nivolumab in 4-week intervals.
8 mg of compound 1 or a salt thereof of the present disclosure once a day and 1 to 4 mg/kg (body weight) per dose of nivolumab.
8 mg of compound 1 or a salt thereof of the present disclosure once a day and 1 to 3 mg/kg (body weight) per dose of nivolumab.
8 mg of compound 1 or a salt thereof of the present disclosure once a day and 2 to 3 mg/kg (body weight) per dose of nivolumab.
8 mg of compound 1 or a salt thereof of the present disclosure once a day and 1 mg/kg (body weight) per dose of nivolumab.
8 mg of compound 1 or a salt thereof of the present disclosure once a day and 2 mg/kg (body weight) per dose of nivolumab.
8 mg of compound 1 or a salt thereof of the present disclosure once a day and 3 mg/kg (body weight) per dose of nivolumab.
8 mg of compound 1 or a salt thereof of the present disclosure once a day and 80 to 240 mg of nivolumab per dose.
8 mg of compound 1 or a salt thereof of the present disclosure once a day and 80 to 480 mg of nivolumab per dose.
8 mg of compound 1 or a salt thereof of the present disclosure once a day and 160 to 480 mg of nivolumab per dose.
8 mg of compound 1 or a salt thereof of the present disclosure once a day and 80 mg of nivolumab per dose.
8 mg of compound 1 or a salt thereof of the present disclosure once a day and 240 mg of nivolumab per dose.
8 mg of compound 1 or a salt thereof of the present disclosure once a day and 480 mg of nivolumab per dose.
8 mg of compound 1 or a salt thereof of the present disclosure once a day and 1 mg/kg of nivolumab in 2-week intervals.
8 mg of compound 1 or a salt thereof of the present disclosure once a day and 2 mg/kg of nivolumab in 2-week intervals.
8 mg of compound 1 or a salt thereof of the present disclosure once a day and 3 mg/kg of nivolumab in 2-week intervals.
8 mg of compound 1 or a salt thereof of the present disclosure once a day and 1 mg/kg of nivolumab in 3-week intervals.
8 mg of compound 1 or a salt thereof of the present disclosure once a day and 2 mg/kg of nivolumab in 3-week intervals.
8 mg of compound 1 or a salt thereof of the present disclosure once a day and 3 mg/kg of nivolumab in 3-week intervals.
8 mg of compound 1 or a salt thereof of the present disclosure once a day and 1 mg/kg of nivolumab in 4-week intervals.
8 mg of compound 1 or a salt thereof of the present disclosure once a day and 2 mg/kg of nivolumab in 4-week intervals.
8 mg of compound 1 or a salt thereof of the present disclosure once a day and 3 mg/kg of nivolumab in 4-week intervals.
8 mg of compound 1 or a salt thereof of the present disclosure once a day and 80 mg of nivolumab in 2-week intervals.
8 mg of compound 1 or a salt thereof of the present disclosure once a day and 240 mg of nivolumab in 2-week intervals.
8 mg of compound 1 or a salt thereof of the present disclosure once a day and 480 mg of nivolumab in 2-week intervals.
8 mg of compound 1 or a salt thereof of the present disclosure once a day and 80 mg of nivolumab in 3-week intervals.
8 mg of compound 1 or a salt thereof of the present disclosure once a day and 240 mg of nivolumab in 3-week intervals.
8 mg of compound 1 or a salt thereof of the present disclosure once a day and 480 mg of nivolumab in 3-week intervals.
8 mg of compound 1 or a salt thereof of the present disclosure once a day and 80 mg of nivolumab in 4-week intervals.
8 mg of compound 1 or a salt thereof of the present disclosure once a day and 240 mg of nivolumab in 4-week intervals.
8 mg of compound 1 or a salt thereof of the present disclosure once a day and 480 mg of nivolumab in 4-week intervals.
8 mg of compound 1 or a salt thereof of the present disclosure once a day and 80 mg of nivolumab four times in 3-week intervals, and then from the fifth time, 240 mg of nivolumab in 2-week intervals.
8 mg of compound 1 or a salt thereof of the present disclosure once a day and 240 mg of nivolumab four times in 3-week intervals, and then from the fifth time, 240 mg of nivolumab in 2-week intervals.
8 mg of compound 1 or a salt thereof of the present disclosure once a day and 80 mg of nivolumab four times in 3-week intervals, and then from the fifth time, 480 mg of nivolumab in 4-week intervals.
8 mg of compound 1 or a salt thereof of the present disclosure once a day and 240 mg of nivolumab four times in 3-week intervals, and then from the fifth time, 480 mg of nivolumab in 4-week intervals.
12 mg of compound 1 or a salt thereof of the present disclosure once a day and 1 to 4 mg/kg (body weight) per dose of nivolumab.
12 mg of compound 1 or a salt thereof of the present disclosure once a day and 1 to 3 mg/kg (body weight) per dose of nivolumab.
12 mg of compound 1 or a salt thereof of the present disclosure once a day and 2 to 3 mg/kg (body weight) per dose of nivolumab.
12 mg of compound 1 or a salt thereof of the present disclosure once a day and 1 mg/kg (body weight) per dose of nivolumab.
12 mg of compound 1 or a salt thereof of the present disclosure once a day and 2 mg/kg (body weight) per dose of nivolumab.
12 mg of compound 1 or a salt thereof of the present disclosure once a day and 3 mg/kg (body weight) per dose of nivolumab.
12 mg of compound 1 or a salt thereof of the present disclosure once a day and 80 to 240 mg of nivolumab per dose.
12 mg of compound 1 or a salt thereof of the present disclosure once a day and 80 to 480 mg of nivolumab per dose.
12 mg of compound 1 or a salt thereof of the present disclosure once a day and 160 to 480 mg of nivolumab per dose.
12 mg of compound 1 or a salt thereof of the present disclosure once a day and 80 mg of nivolumab per dose.
12 mg of compound 1 or a salt thereof of the present disclosure once a day and 240 mg of nivolumab per dose.
12 mg of compound 1 or a salt thereof of the present disclosure once a day and 480 mg of nivolumab per dose.
12 mg of compound 1 or a salt thereof of the present disclosure once a day and 1 mg/kg of nivolumab in 2-week intervals.
12 mg of compound 1 or a salt thereof of the present disclosure once a day and 2 mg/kg of nivolumab in 2-week intervals.
12 mg of compound 1 or a salt thereof of the present disclosure once a day and 3 mg/kg of nivolumab in 2-week intervals.
12 mg of compound 1 or a salt thereof of the present disclosure once a day and 1 mg/kg of nivolumab in 3-week intervals.
12 mg of compound 1 or a salt thereof of the present disclosure once a day and 2 mg/kg of nivolumab in 3-week intervals.
12 mg of compound 1 or a salt thereof of the present disclosure once a day and 3 mg/kg of nivolumab in 3-week intervals.
12 mg of compound 1 or a salt thereof of the present disclosure once a day and 1 mg/kg of nivolumab in 4-week intervals.
12 mg of compound 1 or a salt thereof of the present disclosure once a day and 2 mg/kg of nivolumab in 4-week intervals.
12 mg of compound 1 or a salt thereof of the present disclosure once a day and 3 mg/kg of nivolumab in 4-week intervals.
12 mg of compound 1 or a salt thereof of the present disclosure once a day and 80 mg of nivolumab in 2-week intervals.
12 mg of compound 1 or a salt thereof of the present disclosure once a day and 240 mg of nivolumab in 2-week intervals.
12 mg of compound 1 or a salt thereof of the present disclosure once a day and 480 mg of nivolumab in 2-week intervals.
12 mg of compound 1 or a salt thereof of the present disclosure once a day and 80 mg of nivolumab in 3-week intervals.
12 mg of compound 1 or a salt thereof of the present disclosure once a day and 240 mg of nivolumab in 3-week intervals.
12 mg of compound 1 or a salt thereof of the present disclosure once a day and 480 mg of nivolumab in 3-week intervals.
12 mg of compound 1 or a salt thereof of the present disclosure once a day and 80 mg of nivolumab in 4-week intervals.
12 mg of compound 1 or a salt thereof of the present disclosure once a day and 240 mg of nivolumab in 4-week intervals.
12 mg of compound 1 or a salt thereof of the present disclosure once a day and 480 mg of nivolumab in 4-week intervals.
12 mg of compound 1 or a salt thereof of the present disclosure once a day and 80 mg of nivolumab four times in 3-week intervals, and then from the fifth time, 240 mg of nivolumab in 2-week intervals.
12 mg of compound 1 or a salt thereof of the present disclosure once a day and 240 mg of nivolumab four times in 3-week intervals, and then from the fifth time, 240 mg of nivolumab in 2-week intervals.
12 mg of compound 1 or a salt thereof of the present disclosure once a day and 80 mg of nivolumab four times in 3-week intervals, and then from the fifth time, 480 mg of nivolumab in 4-week intervals.
12 mg of compound 1 or a salt thereof of the present disclosure once a day and 240 mg of nivolumab four times in 3-week intervals, and then from the fifth time, 480 mg of nivolumab in 4-week intervals.
16 mg of compound 1 or a salt thereof of the present disclosure once a day and 1 to 4 mg/kg (body weight) per dose of nivolumab.
16 mg of compound 1 or a salt thereof of the present disclosure once a day and 1 to 3 mg/kg (body weight) per dose of nivolumab.
16 mg of compound 1 or a salt thereof of the present disclosure once a day and 2 to 3 mg/kg (body weight) per dose of nivolumab.
16 mg of compound 1 or a salt thereof of the present disclosure once a day and 1 mg/kg (body weight) per dose of nivolumab.
16 mg of compound 1 or a salt thereof of the present disclosure once a day and 2 mg/kg (body weight) per dose of nivolumab.
16 mg of compound 1 or a salt thereof of the present disclosure once a day and 3 mg/kg (body weight) per dose of nivolumab.
16 mg of compound 1 or a salt thereof of the present disclosure once a day and 80 to 240 mg of nivolumab per dose.
16 mg of compound 1 or a salt thereof of the present disclosure once a day and 80 to 480 mg of nivolumab per dose.
16 mg of compound 1 or a salt thereof of the present disclosure once a day and 160 to 480 mg of nivolumab per dose.
16 mg of compound 1 or a salt thereof of the present disclosure once a day and 80 mg of nivolumab per dose.
16 mg of compound 1 or a salt thereof of the present disclosure once a day and 240 mg of nivolumab per dose.
16 mg of compound 1 or a salt thereof of the present disclosure once a day and 480 mg of nivolumab per dose.
16 mg of compound 1 or a salt thereof of the present disclosure once a day and 1 mg/kg of nivolumab in 2-week intervals.
16 mg of compound 1 or a salt thereof of the present disclosure once a day and 2 mg/kg of nivolumab in 2-week intervals.
16 mg of compound 1 or a salt thereof of the present disclosure once a day and 3 mg/kg of nivolumab in 2-week intervals.
16 mg of compound 1 or a salt thereof of the present disclosure once a day and 1 mg/kg of nivolumab in 3-week intervals.
16 mg of compound 1 or a salt thereof of the present disclosure once a day and 2 mg/kg of nivolumab in 3-week intervals.
16 mg of compound 1 or a salt thereof of the present disclosure once a day and 3 mg/kg of nivolumab in 3-week intervals.
16 mg of compound 1 or a salt thereof of the present disclosure once a day and 1 mg/kg of nivolumab in 4-week intervals.
16 mg of compound 1 or a salt thereof of the present disclosure once a day and 2 mg/kg of nivolumab in 4-week intervals.
16 mg of compound 1 or a salt thereof of the present disclosure once a day and 3 mg/kg of nivolumab in 4-week intervals.
16 mg of compound 1 or a salt thereof of the present disclosure once a day and 80 mg of nivolumab in 2-week intervals.
16 mg of compound 1 or a salt thereof of the present disclosure once a day and 240 mg of nivolumab in 2-week intervals.
16 mg of compound 1 or a salt thereof of the present disclosure once a day and 480 mg of nivolumab in 2-week intervals.
16 mg of compound 1 or a salt thereof of the present disclosure once a day and 80 mg of nivolumab in 3-week intervals.
16 mg of compound 1 or a salt thereof of the present disclosure once a day and 240 mg of nivolumab in 3-week intervals.
16 mg of compound 1 or a salt thereof of the present disclosure once a day and 480 mg of nivolumab in 3-week intervals.
16 mg of compound 1 or a salt thereof of the present disclosure once a day and 80 mg of nivolumab in 4-week intervals.
16 mg of compound 1 or a salt thereof of the present disclosure once a day and 240 mg of nivolumab in 4-week intervals.
16 mg of compound 1 or a salt thereof of the present disclosure once a day and 480 mg of nivolumab in 4-week intervals.
16 mg of compound 1 or a salt thereof of the present disclosure once a day and 80 mg of nivolumab four times in 3-week intervals, and then from the fifth time, 240 mg of nivolumab in 2-week intervals.
16 mg of compound 1 or a salt thereof of the present disclosure once a day and 240 mg of nivolumab four times in 3-week intervals, and then from the fifth time, 240 mg of nivolumab in 2-week intervals.
16 mg of compound 1 or a salt thereof of the present disclosure once a day and 80 mg of nivolumab four times in 3-week intervals, and then from the fifth time, 480 mg of nivolumab in 4-week intervals.
16 mg of compound 1 or a salt thereof of the present disclosure once a day and 240 mg of nivolumab four times in 3-week intervals, and then from the fifth time, 480 mg of nivolumab in 4-week intervals.
20 mg of compound 1 or a salt thereof of the present disclosure once a day and 1 to 4 mg/kg (body weight) per dose of nivolumab.
20 mg of compound 1 or a salt thereof of the present disclosure once a day and 1 to 3 mg/kg (body weight) per dose of nivolumab.
20 mg of compound 1 or a salt thereof of the present disclosure once a day and 2 to 3 mg/kg (body weight) per dose of nivolumab.
20 mg of compound 1 or a salt thereof of the present disclosure once a day and 1 mg/kg (body weight) per dose of nivolumab.
20 mg of compound 1 or a salt thereof of the present disclosure once a day and 2 mg/kg (body weight) per dose of nivolumab.
20 mg of compound 1 or a salt thereof of the present disclosure once a day and 3 mg/kg (body weight) per dose of nivolumab.
20 mg of compound 1 or a salt thereof of the present disclosure once a day and 80 to 240 mg of nivolumab per dose.
20 mg of compound 1 or a salt thereof of the present disclosure once a day and 80 to 480 mg of nivolumab per dose.
20 mg of compound 1 or a salt thereof of the present disclosure once a day and 160 to 480 mg of nivolumab per dose.
20 mg of compound 1 or a salt thereof of the present disclosure once a day and 80 mg of nivolumab per dose.
20 mg of compound 1 or a salt thereof of the present disclosure once a day and 240 mg of nivolumab per dose.
20 mg of compound 1 or a salt thereof of the present disclosure once a day and 480 mg of nivolumab per dose.
20 mg of compound 1 or a salt thereof of the present disclosure once a day and 1 mg/kg of nivolumab in 2-week intervals.
20 mg of compound 1 or a salt thereof of the present disclosure once a day and 2 mg/kg of nivolumab in 2-week intervals.
20 mg of compound 1 or a salt thereof of the present disclosure once a day and 3 mg/kg of nivolumab in 2-week intervals.
20 mg of compound 1 or a salt thereof of the present disclosure once a day and 1 mg/kg of nivolumab in 3-week intervals.
20 mg of compound 1 or a salt thereof of the present disclosure once a day and 2 mg/kg of nivolumab in 3-week intervals.
20 mg of compound 1 or a salt thereof of the present disclosure once a day and 3 mg/kg of nivolumab in 3-week intervals.
20 mg of compound 1 or a salt thereof of the present disclosure once a day and 1 mg/kg of nivolumab in 4-week intervals.
20 mg of compound 1 or a salt thereof of the present disclosure once a day and 2 mg/kg of nivolumab in 4-week intervals.
20 mg of compound 1 or a salt thereof of the present disclosure once a day and 3 mg/kg of nivolumab in 4-week intervals.
20 mg of compound 1 or a salt thereof of the present disclosure once a day and 80 mg of nivolumab in 2-week intervals.
20 mg of compound 1 or a salt thereof of the present disclosure once a day and 240 mg of nivolumab in 2-week intervals.
20 mg of compound 1 or a salt thereof of the present disclosure once a day and 480 mg of nivolumab in 2-week intervals.
20 mg of compound 1 or a salt thereof of the present disclosure once a day and 80 mg of nivolumab in 3-week intervals.
20 mg of compound 1 or a salt thereof of the present disclosure once a day and 240 mg of nivolumab in 3-week intervals.
20 mg of compound 1 or a salt thereof of the present disclosure once a day and 480 mg of nivolumab in 3-week intervals.
20 mg of compound 1 or a salt thereof of the present disclosure once a day and 80 mg of nivolumab in 4-week intervals.
20 mg of compound 1 or a salt thereof of the present disclosure once a day and 240 mg of nivolumab in 4-week intervals.
20 mg of compound 1 or a salt thereof of the present disclosure once a day and 480 mg of nivolumab in 4-week intervals.
20 mg of compound 1 or a salt thereof of the present disclosure once a day and 80 mg of nivolumab four times in 3-week intervals, and then from the fifth time, 240 mg of nivolumab in 2-week intervals.
20 mg of compound 1 or a salt thereof of the present disclosure once a day and 240 mg of nivolumab four times in 3-week intervals, and then from the fifth time, 240 mg of nivolumab in 2-week intervals.
20 mg of compound 1 or a salt thereof of the present disclosure once a day and 80 mg of nivolumab four times in 3-week intervals, and then from the fifth time, 480 mg of nivolumab in 4-week intervals.
20 mg of compound 1 or a salt thereof of the present disclosure once a day and 240 mg of nivolumab four times in 3-week intervals, and then from the fifth time, 480 mg of nivolumab in 4-week intervals.

Examples of the dose of compound 1 or a salt thereof of the present disclosure per day on the day of administration, and the dose of atezolizumab per day, or a preferred combination of usage and dosage include the following:
4 mg to 160 mg of compound 1 or a salt thereof of the present disclosure and 420 to 1680 mg of atezolizumab per dose.
4 mg to 160 mg of compound 1 or a salt thereof of the present disclosure and 840 to 1680 mg of atezolizumab per dose.
4 mg to 160 mg of compound 1 or a salt thereof of the present disclosure and 840 mg of atezolizumab per dose.
4 mg to 160 mg of compound 1 or a salt thereof of the present disclosure and 1200 mg of atezolizumab per dose.
4 mg to 160 mg of compound 1 or a salt thereof of the present disclosure and 1680 mg of atezolizumab per dose.
4 mg to 160 mg of compound 1 or a salt thereof of the present disclosure and 840 mg of atezolizumab in 2-week intervals.
4 mg to 160 mg of compound 1 or a salt thereof of the present disclosure and 1200 mg of atezolizumab in 2-week intervals.
4 mg to 160 mg of compound 1 or a salt thereof of the present disclosure and 1680 mg of atezolizumab in 2-week intervals.
4 mg to 160 mg of compound 1 or a salt thereof of the present disclosure and 840 mg of atezolizumab in 3-week intervals.
4 mg to 160 mg of compound 1 or a salt thereof of the present disclosure and 1200 mg of atezolizumab in 3-week intervals.
4 mg to 160 mg of compound 1 or a salt thereof of the present disclosure and 1680 mg of atezolizumab in 3-week intervals.
4 mg to 24 mg of compound 1 or a salt thereof of the present disclosure and 420 to 1680 mg of atezolizumab per dose.
4 mg to 24 mg of compound 1 or a salt thereof of the present disclosure and 840 to 1680 mg of atezolizumab per dose.
4 mg to 24 mg of compound 1 or a salt thereof of the present disclosure and 840 mg of atezolizumab per dose.
4 mg to 24 mg of compound 1 or a salt thereof of the present disclosure and 1200 mg of atezolizumab per dose.
4 mg to 24 mg of compound 1 or a salt thereof of the present disclosure and 1680 mg of atezolizumab per dose.
4 mg to 24 mg of compound 1 or a salt thereof of the present disclosure and 840 mg of atezolizumab in 2-week intervals.
4 mg to 24 mg of compound 1 or a salt thereof of the present disclosure and 1200 mg of atezolizumab in 2-week intervals.
4 mg to 24 mg of compound 1 or a salt thereof of the present disclosure and 1680 mg of atezolizumab in 2-week intervals.
4 mg to 24 mg of compound 1 or a salt thereof of the present disclosure and 840 mg of atezolizumab in 3-week intervals.
4 mg to 24 mg of compound 1 or a salt thereof of the present disclosure and 1200 mg of atezolizumab in 3-week intervals.
4 mg to 24 mg of compound 1 or a salt thereof of the present disclosure and 1680 mg of atezolizumab in 3-week intervals.
12 to 24 mg of compound 1 or a salt thereof of the present disclosure and 420 to 1680 mg of atezolizumab per dose.
12 to 24 mg of compound 1 or a salt thereof of the present disclosure and 840 to 1680 mg of atezolizumab per dose.
12 to 24 mg of compound 1 or a salt thereof of the present disclosure and 840 mg of atezolizumab per dose.
12 to 24 mg of compound 1 or a salt thereof of the present disclosure and 1200 mg of atezolizumab per dose.
12 to 24 mg of compound 1 or a salt thereof of the present disclosure and 1680 mg of atezolizumab per dose.
12 to 24 mg of compound 1 or a salt thereof of the present disclosure and 840 mg of atezolizumab in 2-week intervals.
12 to 24 mg of compound 1 or a salt thereof of the present disclosure and 1200 mg of atezolizumab in 2-week intervals.
12 to 24 mg of compound 1 or a salt thereof of the present disclosure and 1680 mg of atezolizumab in 2-week intervals.
12 to 24 mg of compound 1 or a salt thereof of the present disclosure and 840 mg of atezolizumab in 3-week intervals.
12 to 24 mg of compound 1 or a salt thereof of the present disclosure and 1200 mg of atezolizumab in 3-week intervals.
12 to 24 mg of compound 1 or a salt thereof of the present disclosure and 1680 mg of atezolizumab in 3-week intervals.
16 to 24 mg of compound 1 or a salt thereof of the present disclosure and 420 to 1680 mg of atezolizumab per dose.
16 to 24 mg of compound 1 or a salt thereof of the present disclosure and 840 to 1680 mg of atezolizumab per dose.
16 to 24 mg of compound 1 or a salt thereof of the present disclosure and 840 mg of atezolizumab per dose.
16 to 24 mg of compound 1 or a salt thereof of the present disclosure and 1200 mg of atezolizumab per dose.
16 to 24 mg of compound 1 or a salt thereof of the present disclosure and 1680 mg of atezolizumab per dose.
16 to 24 mg of compound 1 or a salt thereof of the present disclosure and 840 mg of atezolizumab in 2-week intervals.
16 to 24 mg of compound 1 or a salt thereof of the present disclosure and 1200 mg of atezolizumab in 2-week intervals.
16 to 24 mg of compound 1 or a salt thereof of the present disclosure and 1680 mg of atezolizumab in 2-week intervals.
16 to 24 mg of compound 1 or a salt thereof of the present disclosure and 840 mg of atezolizumab in 3-week intervals.
16 to 24 mg of compound 1 or a salt thereof of the present disclosure and 1200 mg of atezolizumab in 3-week intervals.
16 to 24 mg of compound 1 or a salt thereof of the present disclosure and 1680 mg of atezolizumab in 3-week intervals.

Examples of the number of doses and the dose of compound 1 or a salt thereof of the present disclosure per day on the day of administration, and the dose of atezolizumab per day, or a combination of usage and dosage include the following:
4 mg of compound 1 or a salt thereof of the present disclosure once a day and 420 to 1680 mg of atezolizumab per dose.
4 mg of compound 1 or a salt thereof of the present disclosure once a day and 840 to 1680 mg of atezolizumab per dose.
4 mg of compound 1 or a salt thereof of the present disclosure once a day and 840 mg of atezolizumab per dose.
4 mg of compound 1 or a salt thereof of the present disclosure once a day and 1200 mg of atezolizumab per dose.
4 mg of compound 1 or a salt thereof of the present disclosure once a day and 1680 mg of atezolizumab per dose.
4 mg of compound 1 or a salt thereof of the present disclosure once a day and 840 mg of atezolizumab in 2-week intervals.
4 mg of compound 1 or a salt thereof of the present disclosure once a day and 1200 mg of atezolizumab in 2-week intervals.
4 mg of compound 1 or a salt thereof of the present disclosure once a day and 1680 mg of atezolizumab in 2-week intervals.
4 mg of compound 1 or a salt thereof of the present disclosure once a day and 840 mg of atezolizumab in 3-week intervals.
4 mg of compound 1 or a salt thereof of the present disclosure once a day and 1200 mg of atezolizumab in 3-week intervals.
4 mg of compound 1 or a salt thereof of the present disclosure once a day and 1680 mg of atezolizumab in 3-week intervals.
8 mg of compound 1 or a salt thereof of the present disclosure once a day and 420 to 1680 mg of atezolizumab per dose.
8 mg of compound 1 or a salt thereof of the present disclosure once a day and 840 to 1680 mg of atezolizumab per dose.
8 mg of compound 1 or a salt thereof of the present disclosure once a day and 840 mg of atezolizumab per dose.
8 mg of compound 1 or a salt thereof of the present disclosure once a day and 1200 mg of atezolizumab per dose.
8 mg of compound 1 or a salt thereof of the present disclosure once a day and 1680 mg of atezolizumab per dose.
8 mg of compound 1 or a salt thereof of the present disclosure once a day and 840 mg of atezolizumab in 2-week intervals.
8 mg of compound 1 or a salt thereof of the present disclosure once a day and 1200 mg of atezolizumab in 2-week intervals.
8 mg of compound 1 or a salt thereof of the present disclosure once a day and 1680 mg of atezolizumab in 2-week intervals.
8 mg of compound 1 or a salt thereof of the present disclosure once a day and 840 mg of atezolizumab in 3-week intervals.
8 mg of compound 1 or a salt thereof of the present disclosure once a day and 1200 mg of atezolizumab in 3-week intervals.
8 mg of compound 1 or a salt thereof of the present disclosure once a day and 1680 mg of atezolizumab in 3-week intervals.
12 mg of compound 1 or a salt thereof of the present disclosure once a day and 420 to 1680 mg of atezolizumab per dose.
12 mg of compound 1 or a salt thereof of the present disclosure once a day and 840 to 1680 mg of atezolizumab per dose.
12 mg of compound 1 or a salt thereof of the present disclosure once a day and 840 mg of atezolizumab per dose.
12 mg of compound 1 or a salt thereof of the present disclosure once a day and 1200 mg of atezolizumab per dose.
12 mg of compound 1 or a salt thereof of the present disclosure once a day and 1680 mg of atezolizumab per dose.
12 mg of compound 1 or a salt thereof of the present disclosure once a day and 840 mg of atezolizumab in 2-week intervals.
12 mg of compound 1 or a salt thereof of the present disclosure once a day and 1200 mg of atezolizumab in 2-week intervals.
12 mg of compound 1 or a salt thereof of the present disclosure once a day and 1680 mg of atezolizumab in 2-week intervals.
12 mg of compound 1 or a salt thereof of the present disclosure once a day and 840 mg of atezolizumab in 3-week intervals.
12 mg of compound 1 or a salt thereof of the present disclosure once a day and 1200 mg of atezolizumab in 3-week intervals.
12 mg of compound 1 or a salt thereof of the present disclosure once a day and 1680 mg of atezolizumab in 3-week intervals.
16 mg of compound 1 or a salt thereof of the present disclosure once a day and 420 to 1680 mg of atezolizumab per dose.
16 mg of compound 1 or a salt thereof of the present disclosure once a day and 840 to 1680 mg of atezolizumab per dose.
16 mg of compound 1 or a salt thereof of the present disclosure once a day and 840 mg of atezolizumab per dose.
16 mg of compound 1 or a salt thereof of the present disclosure once a day and 1200 mg of atezolizumab per dose.
16 mg of compound 1 or a salt thereof of the present disclosure once a day and 1680 mg of atezolizumab per dose.
16 mg of compound 1 or a salt thereof of the present disclosure once a day and 840 mg of atezolizumab in 2-week intervals.
16 mg of compound 1 or a salt thereof of the present disclosure once a day and 1200 mg of atezolizumab in 2-week intervals.
16 mg of compound 1 or a salt thereof of the present disclosure once a day and 1680 mg of atezolizumab in 2-week intervals.
16 mg of compound 1 or a salt thereof of the present disclosure once a day and 840 mg of atezolizumab in 3-week intervals.
16 mg of compound 1 or a salt thereof of the present disclosure once a day and 1200 mg of atezolizumab in 3-week intervals.
16 mg of compound 1 or a salt thereof of the present disclosure once a day and 1680 mg of atezolizumab in 3-week intervals.
20 mg of compound 1 or a salt thereof of the present disclosure once a day and 420 to 1680 mg of atezolizumab per dose.
20 mg of compound 1 or a salt thereof of the present disclosure once a day and 840 to 1680 mg of atezolizumab per dose.
20 mg of compound 1 or a salt thereof of the present disclosure once a day and 840 mg of atezolizumab per dose.
20 mg of compound 1 or a salt thereof of the present disclosure once a day and 1200 mg of atezolizumab per dose.
20 mg of compound 1 or a salt thereof of the present disclosure once a day and 1680 mg of atezolizumab per dose.
20 mg of compound 1 or a salt thereof of the present disclosure once a day and 840 mg of atezolizumab in 2-week intervals.
20 mg of compound 1 or a salt thereof of the present disclosure once a day and 1200 mg of atezolizumab in 2-week intervals.
20 mg of compound 1 or a salt thereof of the present disclosure once a day and 1680 mg of atezolizumab in 2-week intervals.
20 mg of compound 1 or a salt thereof of the present disclosure once a day and 840 mg of atezolizumab in 3-week intervals.
20 mg of compound 1 or a salt thereof of the present disclosure once a day and 1200 mg of atezolizumab in 3-week intervals.
20 mg of compound 1 or a salt thereof of the present disclosure once a day and 1680 mg of atezolizumab in 3-week intervals.

Examples of the dose of compound 1 or a salt thereof of the present disclosure per day on the day of administration, and the dose of ipilimumab per day, or a preferred combination of usage and dosage include the following:
4 mg to 160 mg of compound 1 or a salt thereof of the present disclosure and 0.5 to 10 mg/kg (body weight) per dose of ipilimumab
4 mg to 160 mg of compound 1 or a salt thereof of the present disclosure and 1.0 to 10 mg/kg (body weight) per dose of ipilimumab.
4 mg to 160 mg of compound 1 or a salt thereof of the present disclosure and 1.0 mg/kg (body weight) per dose of ipilimumab.
4 mg to 160 mg of compound 1 or a salt thereof of the present disclosure and 3.0 mg/kg (body weight) per dose of ipilimumab.
4 mg to 160 mg of compound 1 or a salt thereof of the present disclosure and 10 mg/kg (body weight) per dose of ipilimumab.
4 mg to 160 mg of compound 1 or a salt thereof of the present disclosure and 1.0 mg/kg (body weight) of ipilimumab in 2-week intervals.
4 mg to 160 mg of compound 1 or a salt thereof of the present disclosure and 1.0 mg/kg (body weight) of ipilimumab in 3-week intervals.
4 mg to 160 mg of compound 1 or a salt thereof of the present disclosure and 3.0 mg/kg (body weight) of ipilimumab in 2-week intervals.
4 mg to 160 mg of compound 1 or a salt thereof of the present disclosure and 3.0 mg/kg (body weight) of ipilimumab in 3-week intervals.
4 mg to 160 mg of compound 1 or a salt thereof of the present disclosure and 10 mg/kg (body weight) of ipilimumab in 2-week intervals.
4 mg to 160 mg of compound 1 or a salt thereof of the present disclosure and 10 mg/kg (body weight) of ipilimumab in 3-week intervals.
4 mg to 24 mg of compound 1 or a salt thereof of the present disclosure and 0.5 to 10 mg/kg (body weight) per dose of ipilimumab
4 mg to 24 mg of compound 1 or a salt thereof of the present disclosure and 1.0 to 10 mg/kg (body weight) per dose of ipilimumab.
4 mg to 24 mg of compound 1 or a salt thereof of the present disclosure and 1.0 mg/kg (body weight) per dose of ipilimumab.
4 mg to 24 mg of compound 1 or a salt thereof of the present disclosure and 3.0 mg/kg (body weight) per dose of ipilimumab.
4 mg to 24 mg of compound 1 or a salt thereof of the present disclosure and 10 mg/kg (body weight) per dose of ipilimumab.
4 mg to 24 mg of compound 1 or a salt thereof of the present disclosure and 1.0 mg/kg (body weight) of ipilimumab in 2-week intervals.
4 mg to 24 mg of compound 1 or a salt thereof of the present disclosure and 1.0 mg/kg (body weight) of ipilimumab in 3-week intervals.
4 mg to 24 mg of compound 1 or a salt thereof of the present disclosure and 3.0 mg/kg (body weight) of ipilimumab in 2-week intervals.
4 mg to 24 mg of compound 1 or a salt thereof of the present disclosure and 3.0 mg/kg (body weight) of ipilimumab in 3-week intervals.
4 mg to 24 mg of compound 1 or a salt thereof of the present disclosure and 10 mg/kg (body weight) of ipilimumab in 2-week intervals.
4 mg to 24 mg of compound 1 or a salt thereof of the present disclosure and 10 mg/kg (body weight) of ipilimumab in 3-week intervals.
12 to 24 mg of compound 1 or a salt thereof of the present disclosure and 0.5 to 10 mg/kg (body weight) per dose of ipilimumab
12 to 24 mg of compound 1 or a salt thereof of the present disclosure and 1.0 to 10 mg/kg (body weight) per dose of ipilimumab.
12 to 24 mg of compound 1 or a salt thereof of the present disclosure and 1.0 mg/kg (body weight) per dose of ipilimumab.
12 to 24 mg of compound 1 or a salt thereof of the present disclosure and 3.0 mg/kg (body weight) per dose of ipilimumab.
12 to 24 mg of compound 1 or a salt thereof of the present disclosure and 10 mg/kg (body weight) per dose of ipilimumab.
12 to 24 mg of compound 1 or a salt thereof of the present disclosure and 1.0 mg/kg (body weight) of ipilimumab in 2-week intervals.
12 to 24 mg of compound 1 or a salt thereof of the present disclosure and 1.0 mg/kg (body weight) of ipilimumab in 3-week intervals.
12 to 24 mg of compound 1 or a salt thereof of the present disclosure and 3.0 mg/kg (body weight) of ipilimumab in 2-week intervals.
12 to 24 mg of compound 1 or a salt thereof of the present disclosure and 3.0 mg/kg (body weight) of ipilimumab in 3-week intervals.
12 to 24 mg of compound 1 or a salt thereof of the present disclosure and 10 mg/kg (body weight) of ipilimumab in 2-week intervals.
12 to 24 mg of compound 1 or a salt thereof of the present disclosure and 10 mg/kg (body weight) of ipilimumab in 3-week intervals.
16 to 24 mg of compound 1 or a salt thereof of the present disclosure and 0.5 to 10 mg/kg (body weight) per dose of ipilimumab
16 to 24 mg of compound 1 or a salt thereof of the present disclosure and 1.0 to 10 mg/kg (body weight) per dose of ipilimumab.
16 to 24 mg of compound 1 or a salt thereof of the present disclosure and 1.0 mg/kg (body weight) per dose of ipilimumab.
16 to 24 mg of compound 1 or a salt thereof of the present disclosure and 3.0 mg/kg (body weight) per dose of ipilimumab.
16 to 24 mg of compound 1 or a salt thereof of the present disclosure and 10 mg/kg (body weight) per dose of ipilimumab.
16 to 24 mg of compound 1 or a salt thereof of the present disclosure and 1.0 mg/kg (body weight) of ipilimumab in 2-week intervals.
16 to 24 mg of compound 1 or a salt thereof of the present disclosure and 1.0 mg/kg (body weight) of ipilimumab in 3-week intervals.
16 to 24 mg of compound 1 or a salt thereof of the present disclosure and 3.0 mg/kg (body weight) of ipilimumab in 2-week intervals.
16 to 24 mg of compound 1 or a salt thereof of the present disclosure and 3.0 mg/kg (body weight) of ipilimumab in 3-week intervals.
16 to 24 mg of compound 1 or a salt thereof of the present disclosure and 10 mg/kg (body weight) of ipilimumab in 2-week intervals.
16 to 24 mg of compound 1 or a salt thereof of the present disclosure and 10 mg/kg (body weight) of ipilimumab in 3-week intervals.

Examples of the number of doses and the dose of compound 1 or a salt thereof of the present disclosure per day on the day of administration, and the dose of ipilimumab per day, or a combination of usage and dosage include the following:
4 mg of compound 1 or a salt thereof of the present disclosure once a day and 0.5 to 10 mg/kg (body weight) per dose of ipilimumab
4 mg of compound 1 or a salt thereof of the present disclosure once a day and 1.0 to 10 mg/kg (body weight) per dose of ipilimumab.
4 mg of compound 1 or a salt thereof of the present disclosure once a day and 1.0 mg/kg (body weight) per dose of ipilimumab.
4 mg of compound 1 or a salt thereof of the present disclosure once a day and 3.0 mg/kg (body weight) per dose of ipilimumab.
4 mg of compound 1 or a salt thereof of the present disclosure once a day and 10 mg/kg (body weight) per dose of ipilimumab.
4 mg of compound 1 or a salt thereof of the present disclosure once a day and 1.0 mg/kg (body weight) of ipilimumab in 2-week intervals.
4 mg of compound 1 or a salt thereof of the present disclosure once a day and 1.0 mg/kg (body weight) of ipilimumab in 3-week intervals.
4 mg of compound 1 or a salt thereof of the present disclosure once a day and 3.0 mg/kg (body weight) of ipilimumab in 2-week intervals.
4 mg of compound 1 or a salt thereof of the present disclosure once a day and 3.0 mg/kg (body weight) of ipilimumab in 3-week intervals.
4 mg of compound 1 or a salt thereof of the present disclosure once a day and 10 mg/kg (body weight) of ipilimumab in 2-week intervals.
4 mg of compound 1 or a salt thereof of the present disclosure once a day and 10 mg/kg (body weight) of ipilimumab in 3-week intervals.
8 mg of compound 1 or a salt thereof of the present disclosure once a day and 0.5 to 10 mg/kg (body weight) per dose of ipilimumab
8 mg of compound 1 or a salt thereof of the present disclosure once a day and 1.0 to 10 mg/kg (body weight) per dose of ipilimumab.
8 mg of compound 1 or a salt thereof of the present disclosure once a day and 1.0 mg/kg (body weight) per dose of ipilimumab.
8 mg of compound 1 or a salt thereof of the present disclosure once a day and 3.0 mg/kg (body weight) per dose of ipilimumab.
8 mg of compound 1 or a salt thereof of the present disclosure once a day and 10 mg/kg (body weight) per dose of ipilimumab.
8 mg of compound 1 or a salt thereof of the present disclosure once a day and 1.0 mg/kg (body weight) of ipilimumab in 2-week intervals.
8 mg of compound 1 or a salt thereof of the present disclosure once a day and 1.0 mg/kg (body weight) of ipilimumab in 3-week intervals.
8 mg of compound 1 or a salt thereof of the present disclosure once a day and 3.0 mg/kg (body weight) of ipilimumab in 2-week intervals.
8 mg of compound 1 or a salt thereof of the present disclosure once a day and 3.0 mg/kg (body weight) of ipilimumab in 3-week intervals.
8 mg of compound 1 or a salt thereof of the present disclosure once a day and 10 mg/kg (body weight) of ipilimumab in 2-week intervals.
8 mg of compound 1 or a salt thereof of the present disclosure once a day and 10 mg/kg (body weight) of ipilimumab in 3-week intervals.
12 mg of compound 1 or a salt thereof of the present disclosure once a day and 0.5 to 10 mg/kg (body weight) per dose of ipilimumab
12 mg of compound 1 or a salt thereof of the present disclosure once a day and 1.0 to 10 mg/kg (body weight) per dose of ipilimumab.
12 mg of compound 1 or a salt thereof of the present disclosure once a day and 1.0 mg/kg (body weight) per dose of ipilimumab.
12 mg of compound 1 or a salt thereof of the present disclosure once a day and 3.0 mg/kg (body weight) per dose of ipilimumab.
12 mg of compound 1 or a salt thereof of the present disclosure once a day and 10 mg/kg (body weight) per dose of ipilimumab.
12 mg of compound 1 or a salt thereof of the present disclosure once a day and 1.0 mg/kg (body weight) of ipilimumab in 2-week intervals.
12 mg of compound 1 or a salt thereof of the present disclosure once a day and 1.0 mg/kg (body weight) of ipilimumab in 3-week intervals.
12 mg of compound 1 or a salt thereof of the present disclosure once a day and 3.0 mg/kg (body weight) of ipilimumab in 2-week intervals.
12 mg of compound 1 or a salt thereof of the present disclosure once a day and 3.0 mg/kg (body weight) of ipilimumab in 3-week intervals.
12 mg of compound 1 or a salt thereof of the present disclosure once a day and 10 mg/kg (body weight) of ipilimumab in 2-week intervals.
12 mg of compound 1 or a salt thereof of the present disclosure once a day and 10 mg/kg (body weight) of ipilimumab in 3-week intervals.
16 mg of compound 1 or a salt thereof of the present disclosure once a day and 0.5 to 10 mg/kg (body weight) per dose of ipilimumab
16 mg of compound 1 or a salt thereof of the present disclosure once a day and 1.0 to 10 mg/kg (body weight) per dose of ipilimumab.
16 mg of compound 1 or a salt thereof of the present disclosure once a day and 1.0 mg/kg (body weight) per dose of ipilimumab.
16 mg of compound 1 or a salt thereof of the present disclosure once a day and 3.0 mg/kg (body weight) per dose of ipilimumab.
16 mg of compound 1 or a salt thereof of the present disclosure once a day and 10 mg/kg (body weight) per dose of ipilimumab.
16 mg of compound 1 or a salt thereof of the present disclosure once a day and 1.0 mg/kg (body weight) of ipilimumab in 2-week intervals.
16 mg of compound 1 or a salt thereof of the present disclosure once a day and 1.0 mg/kg (body weight) of ipilimumab in 3-week intervals.
16 mg of compound 1 or a salt thereof of the present disclosure once a day and 3.0 mg/kg (body weight) of ipilimumab in 2-week intervals.
16 mg of compound 1 or a salt thereof of the present disclosure once a day and 3.0 mg/kg (body weight) of ipilimumab in 3-week intervals.
16 mg of compound 1 or a salt thereof of the present disclosure once a day and 10 mg/kg (body weight) of ipilimumab in 2-week intervals.
16 mg of compound 1 or a salt thereof of the present disclosure once a day and 10 mg/kg (body weight) of ipilimumab in 3-week intervals.
20 mg of compound 1 or a salt thereof of the present disclosure once a day and 0.5 to 10 mg/kg (body weight) per dose of ipilimumab
20 mg of compound 1 or a salt thereof of the present disclosure once a day and 1.0 to 10 mg/kg (body weight) per dose of ipilimumab.
20 mg of compound 1 or a salt thereof of the present disclosure once a day and 1.0 mg/kg (body weight) per dose of ipilimumab.
20 mg of compound 1 or a salt thereof of the present disclosure once a day and 3.0 mg/kg (body weight) per dose of ipilimumab.
20 mg of compound 1 or a salt thereof of the present disclosure once a day and 10 mg/kg (body weight) per dose of ipilimumab.
20 mg of compound 1 or a salt thereof of the present disclosure once a day and 1.0 mg/kg (body weight) of ipilimumab in 2-week intervals.
20 mg of compound 1 or a salt thereof of the present disclosure once a day and 1.0 mg/kg (body weight) of ipilimumab in 3-week intervals.
20 mg of compound 1 or a salt thereof of the present disclosure once a day and 3.0 mg/kg (body weight) of ipilimumab in 2-week intervals.
20 mg of compound 1 or a salt thereof of the present disclosure once a day and 3.0 mg/kg (body weight) of ipilimumab in 3-week intervals.
20 mg of compound 1 or a salt thereof of the present disclosure once a day and 10 mg/kg (body weight) of ipilimumab in 2-week intervals.
20 mg of compound 1 or a salt thereof of the present disclosure once a day and 10 mg/kg (body weight) of ipilimumab in 3-week intervals.

Examples of the dose of compound 1 or a salt thereof of the present disclosure per day on the day of administration, and the dose of durvalumab per day, or a preferred combination of usage and dosage include the following:
4 mg to 160 mg of compound 1 or a salt thereof of the present disclosure and 5.0 to 10 mg/kg (body weight) per dose of durvalumab.
4 mg to 160 mg of compound 1 or a salt thereof of the present disclosure and 10 mg/kg (body weight) per dose of durvalumab.
4 mg to 160 mg of compound 1 or a salt thereof of the present disclosure and 10 mg/kg (body weight) of durvalumab in 2-week intervals.
4 mg to 160 mg of compound 1 or a salt thereof of the present disclosure and 10 mg/kg (body weight) of durvalumab in 3-week intervals.
4 mg to 24 mg of compound 1 or a salt thereof of the present disclosure and 5.0 to 10 mg/kg (body weight) per dose of durvalumab.
4 mg to 24 mg of compound 1 or a salt thereof of the present disclosure and 10 mg/kg (body weight) per dose of durvalumab.
4 mg to 24 mg of compound 1 or a salt thereof of the present disclosure and 10 mg/kg (body weight) of durvalumab in 2-week intervals.
4 mg to 24 mg of compound 1 or a salt thereof of the present disclosure and 10 mg/kg (body weight) of durvalumab in 3-week intervals.
12 to 24 mg of compound 1 or a salt thereof of the present disclosure and 5.0 to 10 mg/kg (body weight) per dose of durvalumab.
12 to 24 mg of compound 1 or a salt thereof of the present disclosure and 10 mg/kg (body weight) per dose of durvalumab.
12 to 24 mg of compound 1 or a salt thereof of the present disclosure and 10 mg/kg (body weight) of durvalumab in 2-week intervals.
12 to 24 mg of compound 1 or a salt thereof of the present disclosure and 10 mg/kg (body weight) of durvalumab in 3-week intervals.
16 to 24 mg of compound 1 or a salt thereof of the present disclosure and 5.0 to 10 mg/kg (body weight) per dose of durvalumab.
16 to 24 mg of compound 1 or a salt thereof of the present disclosure and 10 mg/kg (body weight) per dose of durvalumab.
16 to 24 mg of compound 1 or a salt thereof of the present disclosure and 10 mg/kg (body weight) of durvalumab in 2-week intervals.
16 to 24 mg of compound 1 or a salt thereof of the present disclosure and 10 mg/kg (body weight) of durvalumab in 3-week intervals.

Examples of the number of doses and the dose of compound 1 or a salt thereof of the present disclosure per day on the day of administration, and the dose of durvalumab per day, or a combination of usage and dosage include the following:
4 mg of compound 1 or a salt thereof of the present disclosure once a day and 5.0 to 10 mg/kg (body weight) per dose of durvalumab.
4 mg of compound 1 or a salt thereof of the present disclosure once a day and 10 mg/kg (body weight) per dose of durvalumab.
4 mg of compound 1 or a salt thereof of the present disclosure once a day and 10 mg/kg (body weight) of durvalumab in 2-week intervals.
4 mg of compound 1 or a salt thereof of the present disclosure once a day and 10 mg/kg (body weight) of durvalumab in 3-week intervals.
8 mg of compound 1 or a salt thereof of the present disclosure once a day and 5.0 to 10 mg/kg (body weight) per dose of durvalumab.
8 mg of compound 1 or a salt thereof of the present disclosure once a day and 10 mg/kg (body weight) per dose of durvalumab.
8 mg of compound 1 or a salt thereof of the present disclosure once a day and 10 mg/kg (body weight) of durvalumab in 2-week intervals.
8 mg of compound 1 or a salt thereof of the present disclosure once a day and 10 mg/kg (body weight) of durvalumab in 3-week intervals.
12 mg of compound 1 or a salt thereof of the present disclosure once a day and 5.0 to 10 mg/kg (body weight) per dose of durvalumab.
12 mg of compound 1 or a salt thereof of the present disclosure once a day and 10 mg/kg (body weight) per dose of durvalumab.
12 mg of compound 1 or a salt thereof of the present disclosure once a day and 10 mg/kg (body weight) of durvalumab in 2-week intervals.
12 mg of compound 1 or a salt thereof of the present disclosure once a day and 10 mg/kg (body weight) of durvalumab in 3-week intervals.
16 mg of compound 1 or a salt thereof of the present disclosure once a day and 5.0 to 10 mg/kg (body weight) per dose of durvalumab.
16 mg of compound 1 or a salt thereof of the present disclosure once a day and 10 mg/kg (body weight) per dose of durvalumab.
16 mg of compound 1 or a salt thereof of the present disclosure once a day and 10 mg/kg (body weight) of durvalumab in 2-week intervals.
16 mg of compound 1 or a salt thereof of the present disclosure once a day and 10 mg/kg (body weight) of durvalumab in 3-week intervals.
20 mg of compound 1 or a salt thereof of the present disclosure once a day and 5.0 to 10 mg/kg (body weight) per dose of durvalumab.
20 mg of compound 1 or a salt thereof of the present disclosure once a day and 10 mg/kg (body weight) per dose of durvalumab.
20 mg of compound 1 or a salt thereof of the present disclosure once a day and 10 mg/kg (body weight) of durvalumab in 2-week intervals.
20 mg of compound 1 or a salt thereof of the present disclosure once a day and 10 mg/kg (body weight) of durvalumab in 3-week intervals.

Examples of the dose of compound 1 or a salt thereof of the present disclosure per day on the day of administration, and the dose of avelumab per day, or a preferred combination of usage and dosage include the following:
4 mg to 160 mg of compound 1 or a salt thereof of the present disclosure and 5.0 to 10 g/kg (body weight) per dose of avelumab
4 mg to 160 mg of compound 1 or a salt thereof of the present disclosure and 10 mg/kg (body weight) per dose of avelumab
4 mg to 160 mg of compound 1 or a salt thereof of the present disclosure and 400 to 800 mg of avelumab per dose.
4 mg to 160 mg of compound 1 or a salt thereof of the present disclosure and 800 mg of avelumab per dose.
4 mg to 160 mg of compound 1 or a salt thereof of the present disclosure and 800 mg of avelumab in 2-week intervals.
4 mg to 160 mg of compound 1 or a salt thereof of the present disclosure and 800 mg of avelumab in 3-week intervals.
4 mg to 160 mg of compound 1 or a salt thereof of the present disclosure and 10 mg/kg (body weight) of avelumab in 2-week intervals.
4 mg to 160 mg of compound 1 or a salt thereof of the present disclosure and 10 mg/kg (body weight) of avelumab in 3-week intervals.
4 mg to 24 mg of compound 1 or a salt thereof of the present disclosure and 5.0 to 10 g/kg (body weight) per dose of avelumab
4 mg to 24 mg of compound 1 or a salt thereof of the present disclosure and 10 mg/kg (body weight) per dose of avelumab
4 mg to 24 mg of compound 1 or a salt thereof of the present disclosure and 400 to 800 mg of avelumab per dose.
4 mg to 24 mg of compound 1 or a salt thereof of the present disclosure and 800 mg of avelumab per dose.
4 mg to 24 mg of compound 1 or a salt thereof of the present disclosure and 800 mg of avelumab in 2-week intervals.
4 mg to 24 mg of compound 1 or a salt thereof of the present disclosure and 800 mg of avelumab in 3-week intervals.
4 mg to 24 mg of compound 1 or a salt thereof of the present disclosure and 10 mg/kg (body weight) of avelumab in 2-week intervals.
4 mg to 24 mg of compound 1 or a salt thereof of the present disclosure and 10 mg/kg (body weight) of avelumab in 3-week intervals.
12 to 24 mg of compound 1 or a salt thereof of the present disclosure and 5.0 to 10 g/kg (body weight) per dose of avelumab
12 to 24 mg of compound 1 or a salt thereof of the present disclosure and 10 mg/kg (body weight) per dose of avelumab
12 to 24 mg of compound 1 or a salt thereof of the present disclosure and 400 to 800 mg of avelumab per dose.
12 to 24 mg of compound 1 or a salt thereof of the present disclosure and 800 mg of avelumab per dose.
12 to 24 mg of compound 1 or a salt thereof of the present disclosure and 800 mg of avelumab in 2-week intervals.
12 to 24 mg of compound 1 or a salt thereof of the present disclosure and 800 mg of avelumab in 3-week intervals.
12 to 24 mg of compound 1 or a salt thereof of the present disclosure and 10 mg/kg (body weight) of avelumab in 2-week intervals.
12 to 24 mg of compound 1 or a salt thereof of the present disclosure and 10 mg/kg (body weight) of avelumab in 3-week intervals.
16 to 24 mg of compound 1 or a salt thereof of the present disclosure and 5.0 to 10 g/kg (body weight) per dose of avelumab
16 to 24 mg of compound 1 or a salt thereof of the present disclosure and 10 mg/kg (body weight) per dose of avelumab
16 to 24 mg of compound 1 or a salt thereof of the present disclosure and 400 to 800 mg of avelumab per dose.
16 to 24 mg of compound 1 or a salt thereof of the present disclosure and 800 mg of avelumab per dose.
16 to 24 mg of compound 1 or a salt thereof of the present disclosure and 800 mg of avelumab in 2-week intervals.
16 to 24 mg of compound 1 or a salt thereof of the present disclosure and 800 mg of avelumab in 3-week intervals.
16 to 24 mg of compound 1 or a salt thereof of the present disclosure and 10 mg/kg (body weight) of avelumab in 2-week intervals.
16 to 24 mg of compound 1 or a salt thereof of the present disclosure and 10 mg/kg (body weight) of avelumab in 3-week intervals.

Examples of the number of doses and the dose of compound 1 or a salt thereof of the present disclosure per day on the day of administration, and the dose of avelumab per day, or a combination of usage and dosage include the following:
4 mg of compound 1 or a salt thereof of the present disclosure once a day and 5.0 to 10 g/kg (body weight) per dose of avelumab
4 mg of compound 1 or a salt thereof of the present disclosure once a day and 10 mg/kg (body weight) per dose of avelumab
4 mg of compound 1 or a salt thereof of the present disclosure once a day and 400 to 800 mg of avelumab per dose.
4 mg of compound 1 or a salt thereof of the present disclosure once a day and 800 mg of avelumab per dose.
4 mg of compound 1 or a salt thereof of the present disclosure once a day and 800 mg of avelumab in 2-week intervals.
4 mg of compound 1 or a salt thereof of the present disclosure once a day and 800 mg of avelumab in 3-week intervals.
4 mg of compound 1 or a salt thereof of the present disclosure once a day and 10 mg/kg (body weight) of avelumab in 2-week intervals.
4 mg of compound 1 or a salt thereof of the present disclosure once a day and 10 mg/kg (body weight) of avelumab in 3-week intervals.
8 mg of compound 1 or a salt thereof of the present disclosure once a day and 5.0 to 10 g/kg (body weight) per dose of avelumab
8 mg of compound 1 or a salt thereof of the present disclosure once a day and 10 mg/kg (body weight) per dose of avelumab
8 mg of compound 1 or a salt thereof of the present disclosure once a day and 400 to 800 mg of avelumab per dose.
8 mg of compound 1 or a salt thereof of the present disclosure once a day and 800 mg of avelumab per dose.
8 mg of compound 1 or a salt thereof of the present disclosure once a day and 800 mg of avelumab in 2-week intervals.
8 mg of compound 1 or a salt thereof of the present disclosure once a day and 800 mg of avelumab in 3-week intervals.
8 mg of compound 1 or a salt thereof of the present disclosure once a day and 10 mg/kg (body weight) of avelumab in 2-week intervals.
8 mg of compound 1 or a salt thereof of the present disclosure once a day and 10 mg/kg (body weight) of avelumab in 3-week intervals.
12 mg of compound 1 or a salt thereof of the present disclosure once a day and 5.0 to 10 g/kg (body weight) per dose of avelumab
12 mg of compound 1 or a salt thereof of the present disclosure once a day and 10 mg/kg (body weight) per dose of avelumab
12 mg of compound 1 or a salt thereof of the present disclosure once a day and 400 to 800 mg of avelumab per dose.
12 mg of compound 1 or a salt thereof of the present disclosure once a day and 800 mg of avelumab per dose.
12 mg of compound 1 or a salt thereof of the present disclosure once a day and 800 mg of avelumab in 2-week intervals.
12 mg of compound 1 or a salt thereof of the present disclosure once a day and 800 mg of avelumab in 3-week intervals.
12 mg of compound 1 or a salt thereof of the present disclosure once a day and 10 mg/kg (body weight) of avelumab in 2-week intervals.
12 mg of compound 1 or a salt thereof of the present disclosure once a day and 10 mg/kg (body weight) of avelumab in 3-week intervals.
16 mg of compound 1 or a salt thereof of the present disclosure once a day and 5.0 to 10 g/kg (body weight) per dose of avelumab
16 mg of compound 1 or a salt thereof of the present disclosure once a day and 10 mg/kg (body weight) per dose of avelumab
16 mg of compound 1 or a salt thereof of the present disclosure once a day and 400 to 800 mg of avelumab per dose.
16 mg of compound 1 or a salt thereof of the present disclosure once a day and 800 mg of avelumab per dose.
16 mg of compound 1 or a salt thereof of the present disclosure once a day and 800 mg of avelumab in 2-week intervals.
16 mg of compound 1 or a salt thereof of the present disclosure once a day and 800 mg of avelumab in 3-week intervals.
16 mg of compound 1 or a salt thereof of the present disclosure once a day and 10 mg/kg (body weight) of avelumab in 2-week intervals.
16 mg of compound 1 or a salt thereof of the present disclosure once a day and 10 mg/kg (body weight) of avelumab in 3-week intervals.
20 mg of compound 1 or a salt thereof of the present disclosure once a day and 5.0 to 10 g/kg (body weight) per dose of avelumab
20 mg of compound 1 or a salt thereof of the present disclosure once a day and 10 mg/kg (body weight) per dose of avelumab
20 mg of compound 1 or a salt thereof of the present disclosure once a day and 400 to 800 mg of avelumab per dose.
20 mg of compound 1 or a salt thereof of the present disclosure once a day and 800 mg of avelumab per dose.
20 mg of compound 1 or a salt thereof of the present disclosure once a day and 800 mg of avelumab in 2-week intervals.
20 mg of compound 1 or a salt thereof of the present disclosure once a day and 800 mg of avelumab in 3-week intervals.
20 mg of compound 1 or a salt thereof of the present disclosure once a day and 10 mg/kg (body weight) of avelumab in 2-week intervals.
20 mg of compound 1 or a salt thereof of the present disclosure once a day and 10 mg/kg (body weight) of avelumab in 3-week intervals.

Examples of the dose of compound 1 or a salt thereof of the present disclosure per day on the day of administration, and the dose of cemiplimab per day, or a preferred combination of usage and dosage include the following:
4 mg to 160 mg of compound 1 or a salt thereof of the present disclosure and 175 to 350 mg of cemiplimab per dose.
4 mg to 160 mg of compound 1 or a salt thereof of the present disclosure and 350 mg of cemiplimab per dose.
4 mg to 160 mg of compound 1 or a salt thereof of the present disclosure and 350 mg of cemiplimab in 2-week intervals.
4 mg to 160 mg of compound 1 or a salt thereof of the present disclosure and 350 mg of cemiplimab in 3-week intervals.
4 mg to 24 mg of compound 1 or a salt thereof of the present disclosure and 175 to 350 mg of cemiplimab per dose.
4 mg to 24 mg of compound 1 or a salt thereof of the present disclosure and 350 mg of cemiplimab per dose.
4 mg to 24 mg of compound 1 or a salt thereof of the present disclosure and 350 mg of cemiplimab in 2-week intervals.
4 mg to 24 mg of compound 1 or a salt thereof of the present disclosure and 350 mg of cemiplimab in 3-week intervals.
12 to 24 mg of compound 1 or a salt thereof of the present disclosure and 175 to 350 mg of cemiplimab per dose.
12 to 24 mg of compound 1 or a salt thereof of the present disclosure and 350 mg of cemiplimab per dose.
12 to 24 mg of compound 1 or a salt thereof of the present disclosure and 350 mg of cemiplimab in 2-week intervals.
12 to 24 mg of compound 1 or a salt thereof of the present disclosure and 350 mg of cemiplimab in 3-week intervals.
16 to 24 mg of compound 1 or a salt thereof of the present disclosure and 175 to 350 mg of cemiplimab per dose.
16 to 24 mg of compound 1 or a salt thereof of the present disclosure and 350 mg of cemiplimab per dose.
16 to 24 mg of compound 1 or a salt thereof of the present disclosure and 350 mg of cemiplimab in 2-week intervals.
16 to 24 mg of compound 1 or a salt thereof of the present disclosure and 350 mg of cemiplimab in 3-week intervals.

Examples of the number of doses and the dose of compound 1 or a salt thereof of the present disclosure per day on the day of administration, and the dose of cemiplimab per day, or a combination of usage and dosage include the following:
4 mg of compound 1 or a salt thereof of the present disclosure once a day and 175 to 350 mg of cemiplimab per dose.
4 mg of compound 1 or a salt thereof of the present disclosure once a day and 350 mg of cemiplimab per dose.
4 mg of compound 1 or a salt thereof of the present disclosure once a day and 350 mg of cemiplimab in 2-week intervals.
4 mg of compound 1 or a salt thereof of the present disclosure once a day and 350 mg of cemiplimab in 3-week intervals.
8 mg of compound 1 or a salt thereof of the present disclosure once a day and 175 to 350 mg of cemiplimab per dose.
8 mg of compound 1 or a salt thereof of the present disclosure once a day and 350 mg of cemiplimab per dose.
8 mg of compound 1 or a salt thereof of the present disclosure once a day and 350 mg of cemiplimab in 2-week intervals.
8 mg of compound 1 or a salt thereof of the present disclosure once a day and 350 mg of cemiplimab in 3-week intervals.
12 mg of compound 1 or a salt thereof of the present disclosure once a day and 175 to 350 mg of cemiplimab per dose.
12 mg of compound 1 or a salt thereof of the present disclosure once a day and 350 mg of cemiplimab per dose.
12 mg of compound 1 or a salt thereof of the present disclosure once a day and 350 mg of cemiplimab in 2-week intervals.
12 mg of compound 1 or a salt thereof of the present disclosure once a day and 350 mg of cemiplimab in 3-week intervals.
16 mg of compound 1 or a salt thereof of the present disclosure once a day and 175 to 350 mg of cemiplimab per dose.
16 mg of compound 1 or a salt thereof of the present disclosure once a day and 350 mg of cemiplimab per dose.
16 mg of compound 1 or a salt thereof of the present disclosure once a day and 350 mg of cemiplimab in 2-week intervals.
16 mg of compound 1 or a salt thereof of the present disclosure once a day and 350 mg of cemiplimab in 3-week intervals.
20 mg of compound 1 or a salt thereof of the present disclosure once a day and 175 to 350 mg of cemiplimab per dose.
20 mg of compound 1 or a salt thereof of the present disclosure once a day and 350 mg of cemiplimab per dose.
20 mg of compound 1 or a salt thereof of the present disclosure once a day and 350 mg of cemiplimab in 2-week intervals.
20 mg of compound 1 or a salt thereof of the present disclosure once a day and 350 mg of cemiplimab in 3-week intervals.

The use ratio of compound 1 or a salt thereof to an immune checkpoint inhibitor in the present disclosure is not particularly limited, but can be set so that the amount of the immune checkpoint inhibitor is preferably within a range of 10 to 10000 parts by mass, and more preferably 100 to 1000 parts by mass, based on 100 parts by mass of compound 1 or the salt thereof.

In the present disclosure, the mixing ratio of compound 1 or a salt thereof in a dosage form comprising compound 1 or the salt thereof is not particularly limited, but can be appropriately set within a range of, for example, 100 to 100000 mass%, and preferably 1000 to 10000 mass%. In the present disclosure, the mixing ratio of an immune checkpoint inhibitor in a dosage form comprising the immune checkpoint inhibitor is not particularly limited, but can be appropriately set within a range of, for example, 100 to 100000 mass%, and preferably 1000 to 10000 mass%. In an embodiment in which an antitumor agent is formulated into a plurality of dosage forms, the above-mentioned preferable mixing ratio means the mixing ratio of the active ingredient with respect to the mass of the dosage form comprising the active ingredient, rather than the mixing ratio of the active ingredient with respect to the total amount of the dosage form comprising the active ingredient and the dosage form not comprising the active ingredient. For example, the mixing ratio of compound 1 or a salt thereof means mass% of compound 1 or the salt thereof with respect to the mass of only the dosage form comprising compound 1 or the salt thereof.

In one embodiment of the present disclosure, the combination therapy of the present invention is administered to patients who have not been treated with hormonal therapy, immunotherapy (cancer peptide vaccine therapy, etc.), surgery, radiation therapy, or treatment with chemotherapeutic agents, i.e., treatment-naive patients. In another embodiment, the combination therapy is administered to patients who fail to achieve sustained responses after the previous treatment with chemotherapeutic agents.

In one embodiment of the present disclosure, the combination therapy of the present invention is administered to patients with no treatment experience with immune checkpoint inhibitors. In another embodiment, the combination therapy is administered to patients with treatment experience with FGFR inhibitors.

In one embodiment of the present disclosure, "sustained responses" mean that immune responses to cancer are expanded and maintained by performing immunotherapy using an immune checkpoint inhibitor etc. on the subject. Sustained responses can be detected, for example, by measuring the binding of T-lymphocytes to the immune checkpoint inhibitor.

In the present disclosure, the "recommended dose" is a dose that produces the maximum therapeutic effect within a range that can be safely used without developing serious side effects, determined by clinical trials etc. Specific examples include doses that are described in package inserts, interview forms, treatment guidelines, etc., and that have been approved, recommended, or advised by public institutions or organizations, such as the Japan Pharmaceuticals and Medical Devices Agency (PMDA), the U.S. Food and Drug Administration (FDA), and the European Medicines Agency (EMA). Doses that have been approved by any of the public agencies PMDA, FDA, and EMA are preferable.

The administration schedule of the antitumor agent of the present disclosure can be appropriately selected according to the type of cancer, the stage of cancer, and the like.

In the case of compound 1 or a salt thereof, a daily administration schedule of 1 to 21 days (e.g., 3 weeks (21 days)) is preferred; however, there may be a drug holiday after this schedule, and daily administration for 3 weeks (21 days) is more preferable. The administration schedule of the immune checkpoint inhibitor is preferably 2-week to 4-week intervals, for example. The administration schedule of nivolumab is preferably 2-week to 4-week (e.g., 2-week or 3-week) intervals, and more preferably 3-week intervals. The administration schedule of atezolizumab is preferably 2-week, 3-week, or 4-week intervals, and more preferably 3-week intervals. In the present disclosure, the administration of drug A at intervals of X days means that when the day on which drug A is administered is day 1, the next day is day 2, and the day after is day 3 ... , drug A will be next administered on day X + 1. In addition, when the administration schedule is calculated, "1 week," "2 weeks," "3 weeks," and "4 weeks" mean "7 days," "14 days," "21 days," and "28 days," respectively. Therefore, in the present disclosure, the administration of drug A at intervals of 3 weeks means that when the day on which drug A is administered is day 1, drug A will be next administered on day 22.

The number of daily doses of the antitumor agent of the present disclosure can be appropriately selected according to the type of cancer, the stage of cancer, and the like. In the case of combined administration with nivolumab or atezolizumab, the number of doses is preferably once a day.

The order of administration of compound 1 or a salt thereof and the immune checkpoint inhibitor may be appropriately selected according to the type of cancer, the stage of cancer, and the like; however, in one therapeutic regimen, either of them may be administered first, or both of them may be administered simultaneously.

In one embodiment, compound 1 or a salt thereof enhances the effect of the immune checkpoint inhibitor. In another embodiment, the immune checkpoint inhibitor enhances the action of compound 1 or a salt thereof.

In one embodiment of the present disclosure, although PD-L1 may or may not be expressed, the administration subject is preferably one expressing 1% or more, and more preferably 50% or more, PD-L1.

In one embodiment of the present disclosure, the subject is one having a tumor with a positive test result for PD-L1 expression. PD-L1 expression can be detected using an antihuman PD-L1 antibody for diagnostic purposes, or an antigen-binding fragment thereof, in an IHC assay on FFPE or frozen tissue sections of tumor samples taken from a patient. In general, a doctor is considered to instruct diagnostic tests to determine PD-L1 expression using tumor tissue samples taken from the subject prior to the start of combined administration of an immune checkpoint inhibitor and an FGFR inhibitor; however, it is assumed that the doctor can instruct diagnostic tests before or after the start of treatment, after the start of treatment, e.g., at the end of the treatment cycle.

In the present disclosure, "cancer" or "tumor" refers to the physiological conditions of a mammal characterized by unregulated cell growth. "Cancer" and "tumor" have the same meaning in the present specification and are used interchangeably. Cancers include solid and hematologic cancers. Examples include, but are not limited to, carcinoma, lymphoma, leukemia, blastoma, sarcoma, and borderline malignancy (carcinoid).

Examples of cancers targeted by the combination method of the present disclosure include head and neck cancer, digestive organ cancer (esophageal cancer, gastric cancer, duodenal cancer, liver cancer, biliary tract cancer (e.g., gallbladder and cholangiocarcinoma), pancreatic cancer, small intestine cancer, large intestine cancer (e.g., colorectal cancer, colon cancer, and rectal cancer), etc.), lung cancer (e.g., non-small cell lung cancer and small cell lung cancer), mesothelioma (e.g., malignant pleural mesothelioma, peritoneal mesothelioma, pericardial mesothelioma, and testicular mesothelioma), breast cancer, genital cancer (ovarian cancer, uterine cancer (e.g., cervical cancer, uterine body cancer, and endometrial cancer), etc.), renal cancer, bladder cancer, urothelial cancer, prostate cancer, testicular tumor, skin cancer (e.g., malignant melanoma and epidermal cancer), blood cancer (e.g., multiple myeloma and acute myeloid leukemia), bone and soft tissue tumor, rhabdomyosarcoma, brain tumor, malignant schwannoma, neuroendocrine tumor, thyroid cancer, and the like. In one embodiment, the targets are bladder cancer, urothelial cancer, digestive organ cancer (esophageal cancer, gastric cancer, duodenal cancer, liver cancer, biliary tract cancer (e.g., gallbladder and cholangiocarcinoma), pancreatic cancer, small intestine cancer, large intestine cancer (e.g., colorectal cancer, colon cancer, and rectal cancer), etc.), ovarian cancer, head and neck cancer, and uterine cancer (e.g., cervical cancer and uterine body cancer). The cancers mentioned herein include not only the primary lesion, but also cancer metastasized to other organs (e.g., liver). The antitumor agent of the present disclosure may also be used in postoperative adjuvant chemotherapy performed in order to prevent recurrence after surgical removal of the tumor, or may be used in preoperative adjuvant chemotherapy performed in order to surgically remove the tumor.

In one embodiment of the present disclosure, examples of cancers targeted by the combination method include malignant melanoma, non-small cell lung cancer, Hodgkin's lymphoma, gastric cancer, renal cell cancer, head and neck cancer, malignant pleural mesothelioma, esophageal cancer, and urothelial cancer.

As used in the present specification, the term "combination (therapy)" is intended to define a therapy that includes the use of a combination of two or more compounds/drugs (as defined above). Thus, "combination (therapy)," "combination," and the use of compounds/drugs "in combination" in the present application may mean compounds/drugs administered as part of the same overall therapeutic regimen. The dose of each of the two or more compounds/drugs may be different: each may be administered simultaneously or at different times. Therefore, it is understood that the compounds/drugs of the combination may be administered sequentially (e.g., before or after) or simultaneously, either in the same pharmaceutical formulation (i.e., together) or in different pharmaceutical formulations (i.e., separately). The same formulation is simultaneously a single formulation. On the other hand, different pharmaceutical formulations are non-integral.

In the present specification, the terms "regimen" and "therapeutic regimen" refer to a plan that shows, in chronological order, the type and amount of drugs, duration, procedure, etc., in drug treatment, and that shows the dose, administration method, administration order, and administration day of each drug. The treatment in one therapeutic regimen is, for example, a method that starts administration of drugs to be combined, simultaneously or substantially simultaneously on the first day of the cycle. The treatment in one therapeutic regimen also includes, for example, one cycle with three weeks in which administration of drug A is started first, and administration of drug B is started one week later; however, both drugs are substantially simultaneously administered as one cycle.

The therapeutic regimen of compound 1 or a salt thereof and an immune checkpoint inhibitor of the present disclosure may include not only compound 1 or the salt thereof and the immune checkpoint inhibitor, but also other drugs.

The administration form of the antitumor agent of the present disclosure is not limited, and can be appropriately selected depending on the therapeutic purpose. Specific examples thereof include oral preparations (tablets, coated tablets, powders, granules, capsules, solutions, etc.), injections, suppositories, patches, ointments, and the like. In the case of compound 1 or a salt thereof, oral preparations are preferable. In the case of an anti-PD-1 antibody, an anti-PD-Ll antibody, or an anti-CTLA-4 antibody, the above-mentioned administration forms can be used, and injections are preferable.

According to the antitumor agent of the present disclosure, an immune checkpoint inhibitor and compound 1 or a salt thereof, which are active ingredients, may be directly used as an antitumor agent. Depending on the administration form thereof, a pharmaceutically acceptable carrier may be used to prepare a pharmaceutical composition by a generally known method. Examples of such carriers include various carriers commonly used in general drugs, such as excipients, binders, disintegrants, lubricants, diluents, solubilizing agents, suspending agents, isotonizing agents, pH adjusters, buffers, stabilizers, coloring agents, flavoring agents, and the like.

The antitumor agent of the present disclosure may be formulated into a plurality of dosage forms, or may be formulated into a single dosage form, based on the administration form and administration schedule of each active ingredient. Further, formulations may be produced and sold in a single package suitable for combined administration, or formulations may be produced and sold in separate packages. The same applies to the embodiments of the pharmaceutical composition. Accordingly, the "pharmaceutical composition comprising an immune checkpoint inhibitor and compound 1 or a salt thereof as active ingredients" includes a pharmaceutical composition prepared by separately formulating the active ingredients in a plurality of dosage forms, and a pharmaceutical composition prepared by formulating the active ingredients in a single dosage form. The pharmaceutical composition prepared by separately formulating the active ingredients in a plurality of dosage forms includes a pharmaceutical composition prepared by formulating formulations in a single package suitable for combined administration, and a pharmaceutical composition prepared by formulating formulations in separate packages.

The present disclosure relates to a kit preparation comprising an antitumor agent comprising compound 1 or a salt thereof, and an instruction manual stating that compound 1 or the salt thereof is administered to cancer patients in combination with an immune checkpoint inhibitor. The "instruction manual" as used herein may be one stating the above-mentioned dose. Regardless of whether it is legally binding, an instruction manual that recommends the above-mentioned dose is preferable. Specific examples include package inserts, brochures, and the like. The kit preparation comprising an instruction manual may be one in which the instruction manual is printed or attached to the package of the kit preparation, or may be one in which the instruction manual is enclosed together with an antitumor agent in the package of the kit preparation.

Examples of methods for detecting the mechanism of action by FGFR inhibitors and the expression of resistance to immune checkpoint inhibitors include, but are not limited to, a method for examining the modulation of the tumor immune microenvironment (NPL 13).

The microenvironment of the cancer is important for the efficacy of immune checkpoint inhibitors, and cancer-associated fibroblasts (CAFs) are known to play an important role. FGFs are factors that regulate the growth of fibroblast cells, and their receptors, FGFRs, are molecules that receive signals from FGFs in cells and promote cell growth. Myeloid-derived suppressor cells (MDSC) are immature myeloid cells that increase in tumor tissues, lymph nodes, and peripheral blood under pathological conditions, and are known to have potent immunosuppressive activity.

It has been reported that MDSC can be used as a predictive marker for the effect of immune checkpoint inhibitors and can contribute to patient resistance to immune checkpoint inhibitors (NPL 14).

In the present specification, the resistance (also referred to as tolerance or intractableness) to immune checkpoint inhibitors includes subject conditions in which no therapeutic effect is expected after administration of an immune checkpoint inhibitor, and in which progression of disease is confirmed in at least one clinical evaluation; and subject conditions in which therapeutic effects of immune checkpoint inhibitors cannot be expected, although the subject does not have intolerance to immune checkpoint inhibitors, recurrence after administration of immune checkpoint inhibitors, or treatment experience with immune checkpoint inhibitors. In one embodiment, it is also included that no effective effects are exhibited for immune checkpoint inhibitors in pharmacological tests, such as responses of cell lines that are not considered to show efficacy. Resistance includes natural resistance that an anticancer agent does not show efficacy from the beginning of the treatment, and acquired resistance that an anticancer agent that has initially been effective becomes ineffective as the treatment is continued and leads to the exacerbation of the cancer. In the present specification, resistance includes the meaning of both natural resistance and acquired resistance.

In the present specification, refractory to immune checkpoint inhibitors means that an immune checkpoint inhibitor become ineffective and exhibits no effect after administration of the immune checkpoint inhibitor. One of the causes of refractory is considered to be due to resistance.

The antitumor agent of the present disclosure can be used for the treatment of cancer. The "antitumor effect" when referring to a cancer patient who receives treatment with a therapeutic regimen, such as the combination therapy described in the present specification, means at least one evaluation of, for example, PFS (progression-free survival), DCR (disease control rate), DOR (duration of response), OS (overall survival), ORR (objective response rate), DCR (disease control rate), TTR (time to response), PROs (patient-reported outcomes), and the like. In one embodiment, tumor evaluation for the combination therapy described in the present specification for solid tumors is based on the RECIST 1.1 criteria (Response Evaluation Criteria in Solid Tumors), and the antitumor effect is expressed by SD (stable disease), PR (partial response), CR (complete response), and PD (progressive disease). Tumor evaluation of brain tumors can be carried out by standard brain tumor MRI, including pre- and post-enhancement MRI, using a Gd-MRI (gadolinium (Gd)) chelate contrast agent.

### Examples

The present disclosure is explained in more detail below with reference to Examples and a Reference Example; however, the present disclosure is not limited to these Examples, and many modifications can be made by a person who has ordinary knowledge in this field within the technical idea of the present disclosure.

### Reference Example: Clinical trial for cancer immunotherapy-resistant cancer (e.g., non-small cell lung cancer, esophageal cancer, and urothelial cancer) patients by combined use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and pembrolizumab

This study evaluates the efficacy and safety of the combined use of compound 1 or a salt thereof and pembrolizumab in FGFR-overexpressing cancer patients (with, e.g., non-small cell lung cancer or esophageal cancer), based on the clinical trial registered as JapicCTI-195063 with the Japan Pharmaceutical Information Center. Specifically, administration is performed for a predetermined period of time (e.g., 24 weeks). Compound 1 or a salt thereof is orally administered to the patients at various doses (e.g., 20 mg) for 21 days, and pembrolizumab (e.g., 200 mg per administration) is administered by intravenous drip infusion every three weeks (every 21 days). One cycle is 21 days, and this is repeated. In some cases, a placebo may be used as a control, and compound 1 or a salt thereof and/or pembrolizumab may be additionally administered to other patients. Moreover, combined administration with compound 1 or a salt thereof may be performed on patients who have treatment experience with pembrolizumab.

The combined use of compound 1 or a salt thereof and pembrolizumab can provide therapeutic effects that cannot be expected from monotherapies separately using each of them. For example, in an embodiment of the present disclosure, it can be found that this combined use has a higher degree of effects than at least one of the measurement results of treatment with either of them. Moreover, for example, in an embodiment of the present disclosure, a synergistic effect can be obtained by the combined use of compound 1 or a salt thereof and pembrolizumab.

The combined use of compound 1 or a salt thereof and pembrolizumab can be more effective than the single use of either of them, according to at least one of the following measurement results: reduced number of cancer cells, regression of tumor size, reduced rate of cancer cell infiltration into peripheral organs, reduced rate of tumor metastasis or tumor growth, overall response rate, and extended progression-free survival or overall survival.

### Example 1: Influence on tumor immunity in model subcutaneously implanted with mouse breast cancer cell 4T1

Mouse breast cancer cell line 4T1 was subcutaneously implanted in a 6-week-old male BALB/cAJcl mouse. The day of cell implantation was set to Day 0, and compound 1 was orally administered daily from the next day (Day 1) for 14 days at a dose of 15 mg/kg/day. On the day following the final administration (Day 15), lymphocyte fractions were isolated from the tumor, spleen, and peripheral blood, and myeloid-derived suppressor cells (MDSCs) (CD11b⁺/Gr-1⁺ cells), CD4-positive T cells, and CD8-positive T cells were measured using a cell sorter. Moreover, the production of the model subcutaneously implanted with mouse breast cancer cell 4T1, drug administration, and the measurement of each cell were performed in the same manner as described above, except that the dose of compound 1 was changed to 30 mg/kg/day. As a control, the same operation as described above was performed, except that compound 1 was not administered. Fig. 1 shows the results.

The administration of compound 1 at any dose resulted in a significant reduction in MDSCs in the spleen lymphocyte fractions (Student's t-test, P<0.01), compared with the non-drug administration control. MDSCs in the peripheral blood lymphocyte fractions tended to decrease in a dose-dependent manner compared with the control. The CD4-positive T cells in the peripheral blood lymphocyte fractions, and the CD8-positive T cells in the spleen lymphocyte fractions and peripheral blood lymphocyte fractions, tended to increase in a dose-dependent manner compared with the control.

These results confirmed the inhibitory effect of compound 1 on MDSCs in the in vivo models, and thus suggested the possibility that compound 1 had an antitumor effect via an immunosuppression mechanism. Further, since CD4-positive T cells and CD8-positive T cells increased due to the administration of compound 1, the activation of immune activity in the cancer-bearing mice by compound 1 was confirmed. The above results suggested that the combined use of compound 1 or a salt thereof with an immune checkpoint inhibitor enhances the antitumor effect.

Although 4T1 used in Example 1 is breast cancer cells that have no FGFR genetic aberration, the administration of compound 1 confirmed the inhibitory effect on MDSCs, as well as increased CD4-positive T cells and CD8-positive T cells, indicating that compound 1 has an antitumor effect via an immunosuppression mechanism. Moreover, the administration of vofatamab, which is an anti-FGFR3 antibody, to urothelial cancer patients causes the cancer to change from a "cold" state (low sensitivity to immune checkpoint inhibitors) to a "hot" state (high sensitivity to immune checkpoint inhibitors); thus, response by combined use with pembrolizumab is observed even in patients without FGFR genetic aberration (W. Choi et al., presentation at AACR Special Conference on Bladder Cancer: Transforming the Field, May 18-21, 2019, Denver, USA).

Example 2: Clinical phase I trial for patients with solid cancer by combined use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and nivolumab

This study evaluates the efficacy and safety of the combined use of compound 1 or a salt thereof and nivolumab in patients with solid cancer.

Administration is performed for a predetermined period of time (e.g., 24 weeks). Compound 1 or a salt thereof is orally administered to the patients at various doses (8 mg, 12 mg, 16 mg, and 20 mg) for 21 days, and nivolumab (240 mg per administration) is administered by intravenous drip infusion every two weeks (every 14 days). Alternatively, administration is performed for a predetermined period of time (e.g., 24 weeks). Compound 1 or a salt thereof is orally administered to the patients at various doses (8 mg, 12 mg, 16 mg, and 20 mg) for 21 days, nivolumab (80 mg per administration) is administered by intravenous drip infusion every three weeks (every 21 days) four times, and then nivolumab (240 mg per administration) is administered by intravenous drip infusion every two weeks (every 14 days) from the fifth time. Alternatively, administration is performed for a predetermined period of time (e.g., 24 weeks). Compound 1 or a salt thereof is orally administered to the patients at various doses (8 mg, 12 mg, 16 mg, and 20 mg) for 21 days, nivolumab (240 mg per administration) is administered by intravenous drip infusion every three weeks (every 21 days) four times and then from the fifth time every two weeks (every 14 days), and 21-day administration of compound 1 is repeated. In some cases, compound 1 or a salt thereof and/or nivolumab may be additionally administered to other patients. Moreover, combined administration with compound 1 or a salt thereof may be performed on patients who have treatment experience with nivolumab.

The combined use of compound 1 or a salt thereof and nivolumab can provide therapeutic effects that cannot be expected from monotherapies separately using each of them. For example, in an embodiment of the present disclosure, it can be found that this combined use has a higher degree of effects than at least one of the measurement results of treatment with either of them. Moreover, for example, in an embodiment of the present disclosure, a synergistic effect can be obtained by the combined use of compound 1 or a salt thereof and nivolumab.

The combined use of compound 1 or a salt thereof and nivolumab can be more effective than the single use of either of them, according to at least one of the following measurement results: reduced number of cancer cells, regression of tumor size, reduced rate of cancer cell infiltration into peripheral organs, reduced rate of tumor metastasis or tumor growth, overall response rate, and extended progression-free survival or overall survival.

## Claims

1. An antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient, the antitumor agent being administered in combination with an immune checkpoint inhibitor, except for pembrolizumab, to a cancer patient with resistance to immune checkpoint inhibitors.

2. The antitumor agent according to claim 1, wherein the immune checkpoint inhibitor is at least one member selected from the group consisting of a PD-1 pathway antagonist, an ICOS pathway agonist, a CTLA-4 pathway antagonist, and a CD28 pathway agonist.

3. The antitumor agent according to claim 1 or 2, wherein the immune checkpoint inhibitor is a PD-1 pathway antagonist.

4. The antitumor agent according to claim 2 or 3, wherein the PD-1 pathway antagonist is at least one member selected from the group consisting of an anti-PD-1 antibody, an anti-PD-Ll antibody, and an anti-PD-L2 antibody.

5. The antitumor agent according to any one of claims 2 to 4, wherein the PD-1 pathway antagonist is an anti-PD-1 antibody.

6. The antitumor agent according to claim 4 or 5, wherein the anti-PD-1 antibody is nivolumab, cemiplimab, spartalizumab, tislelizumab, BI754091, dostarlimab, sasanlimab, MGA-012, cetrelimab, AGEN-2034, zimberelimab, camrelizumab, budigalimab, or balstilimab.

7. The antitumor agent according to claim 6, wherein the anti-PD-1 antibody is nivolumab.

8. The antitumor agent according to any one of claims 2 to 7, wherein the PD-1 pathway antagonist is an anti-PD-Ll antibody.

9. The antitumor agent according to claim 8, wherein the anti-PD-Ll antibody is atezolizumab, durvalumab, or avelumab.

10. The antitumor agent according to claim 2, wherein the CTLA-4 pathway antagonist is an anti-CTLA-4 antibody.

11. The antitumor agent according to claim 10, wherein the anti-CTLA-4 antibody is ipilimumab or tremelimumab.

12. The antitumor agent according to any one of claims 1 to 11, wherein a tumor has an aberration in the FGFR pathway.

13. The antitumor agent according to claim 12, wherein the aberration in the FGFR pathway is at least one member selected from the group consisting of FGFR overexpression, FGFR genetic aberration, and aberrant FGFR signaling.

14. The antitumor agent according to any one of claims 1 to 13, wherein treatment with the antitumor agent results in a sustained response in an individual after cessation of the treatment.

15. The antitumor agent according to any one of claims 1 to 14, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is used before, simultaneously with, or after the immune checkpoint inhibitor.

16. The antitumor agent according to any one of claims 1 to 15, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is used continuously or intermittently.

17. The antitumor agent according to any one of claims 1 to 16, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and the immune checkpoint inhibitor are administered in treatment with one therapeutic regimen.

18. The antitumor agent according to any one of claims 1 to 7 and 12 to 17, wherein for a tumor with resistance to immune checkpoint inhibitors, the antitumor agent is used to administer (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof once a day at a dose selected from the group consisting of 4 mg, 8 mg, 12 mg, 16 mg, and 20 mg, and to administer nivolumab at a dose selected from the group consisting of 1 mg/kg in 3-week intervals, 2 mg/kg in 3-week intervals, 3 mg/kg in 3-week intervals, 80 mg in 3-week intervals, 240 mg in 3-week intervals, 1 mg/kg in 2-week intervals, 2 mg/kg in 2-week intervals, 3 mg/kg in 2-week intervals, 80 mg in 2-week intervals, and 240 mg in 2-week intervals.

19. The antitumor agent according to claim 18, wherein for a tumor with resistance to immune checkpoint inhibitors, the antitumor agent is used to administer (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof once a day at a dose selected from the group consisting of 8 mg, 12 mg, 16 mg, and 20 mg, and to administer nivolumab at a dose selected from the group consisting of 240 mg in 2-week intervals and 240 mg in 3-week intervals.

20. The antitumor agent according to claim 18, wherein for a tumor with resistance to immune checkpoint inhibitors, the antitumor agent is used to administer (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof once a day at a dose selected from the group consisting of 8 mg, 12 mg, 16 mg, and 20 mg, and to administer nivolumab at a dose of 240 mg in 2-week intervals.

21. The antitumor agent according to claim 18, wherein for a tumor with resistance to immune checkpoint inhibitors, the antitumor agent is used to administer (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof once a day at a dose selected from the group consisting of 4 mg, 8 mg, 12 mg, 16 mg, and 20 mg, and to administer nivolumab at a dose of 80 mg four times in 3-week intervals and then from the fifth time 240 mg in 2-week intervals.
